# EUROPÄISCHE PATENTSCHRIFT

(11) **EP 0 944 385 B1**
(45) Veröffentlichungstag und Bekanntmachung des Hinweises auf die Patenterteilung: **16.10.2002**
(21) Anmeldenummer: 97954350.1
(22) Anmeldetag: 03.12.1997
(51) Int. Cl.: A61K 31/00, A61P 1/04

(54) **VERWENDUNG VON 7-(1-AMINOMETHYL-2-OXA-7-AZA-BICYCLO 3.3.0]OCT-7-YL)-CHINOLONCARBONSÄURE- UND -NAPHTHYRIDONCARBONSÄURE-DERIVATEN ZUR THERAPIE VON HELICOBACTER-PYLORI-INFEKTIONEN UND DEN DAMIT ASSOZIIERTEN GASTRODUODENALEN ERKRANKUNGEN**
USE OF 7-(1-AMINOMETHYL-2-OXA-7-AZA-BICYCLO 3.3.0]OCT-7-YL)-QUINOLONE CARBOXYLIC ACID AND NAPHTHYRIDONE CARBOXYLIC ACID DERIVATIVES FOR TREATING HELICOBACTER PYLORI INFECTIONS AND THE GASTRODUODENAL DISEASES ASSOCIATED THEREWITH
UTILISATION DE DERIVES DE 7-(1-AMINOMETHYLE-2-OXA-7-AZA-BICYCLO 3.3.0]OCT-7-YLE)]-ACIDE CARBOXYLIQUE DE QUINOLONE ET DE 7-(1-AMINOMETHYLE-2-OXA-7-AZA-BICYCLO 3.3.0]OCT-7-YLE)]-ACIDE CARBOXYLIQUE DE NAPHTHYRIDONE POUR TRAITER DES INFECTIONS A HELICOBACTER PYLORI ET DES AFFECTIONS GASTRO-DUODENALES QU

(30) Priorität: 16.12.1996 DE 19652219
(43) Veröffentlichungstag der Anmeldung: 29.09.1999
(73) Patentinhaber: BAYER AG, 51368 Leverkusen (DE)
(72) Erfinder: PETERSEN, Uwe, D-51375 Leverkusen (DE); MATZKE, Michael, D-42113 Wuppertal (DE); JAETSCH, Thomas, D-50668 Köln (DE); SCHENKE, Thomas, D-51469 Bergisch Gladbach (DE); HIMMLER, Thomas, D-51519 Odenthal (DE); BARTEL, Stephan, D-51465 Bergisch Gladbach (DE); BAASNER, Bernd, D-51467 Bergisch Gladbach (DE); WERLING, Hans-Otto, D-42113 Wuppertal (DE); SCHALLER, Klaus, D-42109 Wuppertal (DE); LABISCHINSKI, Harald, D-42113 Wuppertal (DE); ENDERMANN, Rainer, D-42113 Wuppertal (DE)
(86) Internationale Anmeldenummer: EP9706751
(87) Internationale Veröffentlichungsnummer: WO98026768

(56) Entgegenhaltungen:
- EP-A- 0 589 318
- EP-A- 0 671 391
- WO-A-97/31919

## Beschreibung

Die Erfindung betrifft die Verwendung von Chinolon- und Naphthyridoncarbonsäure-Derivaten, die in 7-Stellung durch einen 1-Aminomethyl-2-oxa-7-aza-bicyclo[3.3.0]oct-7-yl-Rest substituiert sind, sowie ihre Salze zur Herstellung von Arzneimitteln für die Therapie von Helicobacter-pylori-Infektionen und den damit assoziierten gastroduodenalen Erkrankungen.

Mit der Wiederentdeckung von *Helicobacter pylori* (H. pylori; früher Campylo-bacter pylori) durch Warren und Marshall 1983 konnten in den darauffolgenden Jahren die pathophysiologischen Vorstellungen über die Entstehung gastro-duodenaler Erkrankungen des Menschen grundlegend weiterentwickelt werden.

H. pylori gilt als Auslöser der Typ B-Gastritis und scheint eine ursächliche Rolle in der Perpetuierung der peptischen Ulkuserkrankung zu spielen. Epidemiolo-gische und pathologische Untersuchungen weisen ebenfalls auf einen Zusammen- hang zwischen Langzeitkolonisierung der Magenmucosa mit dem Bakterium und der Entstehung bestimmter Formen des Magenkarzinoms hin. Deshalb wurde H. pylori im Jahre 1994 zum Karzinogen erster Klasse (gefährlichste Krebsauslöser-Kategorie) eingestuft. Ein seltener Magenkrebs, das MALT-Lymphom (mucosa-associated lymphoid tissue), scheint ebenfalls häufig durch den Keim verursacht zu sein. In ersten Kasuistiken verschwanden tatsächlich nach H. pylori-Eradikation nicht nur die reaktiven Infiltrate, sondern auch ein Teil der niedrig malignen MALT-Lymphome. Auch werden Zusammenhänge mit der Riesenfalten-gastritis diskutiert. Die Rolle von H. pylori beim Reizmagen (nichtulzerösen Dyspepsie) ist noch unklar.

Verschiedene epidemiologische Studien kommen zu dem Ergebnis, daß circa die Hälfte der Weltbevölkerung mit dem Bakterium infiziert ist. Die Walirschein- lichkeit der Besiedlung des Magens mit Helicobacter steigt mit dem Lebensalter. Die optimale Anpassung von Helicobacter an die Lebensbedingungen in dem außergewöhnlichen, konkurrenzarmen Habitat Magen scheint die Voraussetzung für die erfolgreiche Etablierung der chronischen Infektion und für die weite Verbreitung dieser pathogenen Spezies zu sein.

Die Erreger sind mit ihren Geißeln nicht nur im flüssigen Milieu, sondern auch im viskösen Mukus der Magenschleimhaut sehr beweglich, adhärieren an die Magenepithelzellen und vermehren sich am besten bei einem Sauerstoffgehalt von 5 %, wie er im Schleim der Magenwand herrscht. Außerdem bilden die Bakterien große Mengen des Enzyms Urease, das Harnstoff in Ammoniak und Kohlendioxid spaltet. Möglicherweise hilft ihnen die entstehende 'Ammoniakwolke', das saure Milieu in der Mikroumgebung zu neutralisieren und sich so vor der aggressiven Magensäure zu schützen.

### Peptische Ulkuserkrankung

Die Einführung der Histamin-H₂-Rezeptorantagonisten in den 70er Jahren stellte einen Meilenstein in der Therapie der peptischen Ulkuserkrankung dar. Die Häufigkeit chirurgischer Eingriffe zur Behandlung des Ulkusleidens nahm damit weltweit dramatisch ab. Dieses Prinzip der Säureblockade wurde durch die Entwicklung der noch stärker wirksamen Protonenpumpeninhibitoren noch weiter verbessert.

Durch die säurehemmende Therapie können allerdings nur die Symptome der Ulkuserkrankung, nicht der natürliche Verlauf der Erkrankung, der durch das Auftreten von Rezidiven gekennzeichnet ist, kausal - sprich durch keimabtötende Behandlung - beeinflußt werden. Denn praktisch alle Ulcus-duodeni-Patienten und die überwiegende Mehrzahl der Patienten mit Ulcus ventriculi weisen eine H. pylori-Infektion des Magens auf und leiden somit an Infektionskrankheiten. Nur Ulzerationen, die durch nichtsteroidale Antiphlogistika hervorgerufen werden, sind nicht mit einer H. pylori-Infektion assoziiert.

Deshalb sollten nach den Empfehlungen einer Konsensus-Konferenz, die im Jahre 1994 von der amerikanischen Gesundheitsbehörde (NIH) veranstaltet wurde, bei positivem Keimnachweis alle Patienten mit peptischen Ulcera einer gegen H. pylori gerichteten Eradikationstherapie zugeführt werden (NIH Consensus Statement 1: 1-23; 1994). Die Argumente dazu lieferten kontrollierte Therapiestudien, in denen gezeigt werden konnte, daß nach erfolgreicher Keimeradikation die Ulkusrezidivraten drastisch sinken ( 0%- 29% versus 61% - 95%).

### H. pylori-Therapie

Die derzeitige Eradikation des H. pylori gestaltet sich in der Praxis nicht ganz einfach. Eine einfache und dennoch verläßlich wirksame Therapie steht nicht zur Verfügung. Der Keim findet sich gut geschützt und schwer angreifbar unter der Mukusschicht.

H. pylori ist in vitro gegenüber zahlreichen Antibiotika empfindlich. Diese sind allerdings in vivo als Monotherapie nicht effektiv. Dazu zählen u. a. Penicillin, Amoxicillin, Tetracyclin, Erythromycin, Ciprofloxacin, Metronidazol und Clarithromycin. Auch Wismutsalze und im geringen Maße sogar Protonenpumpeninhibitoren (Omeprazol, Lansoprazol) sind in vitro, nicht aber in vivo antibakteriell wirksam.

Unter allen bisher zur Eradikation von H. pylori angewandten Therapiemodalitä-ten sind derzeit nur die folgenden Tripel-Therapien ausreichend wirksam:
1. klassische Wismut-Tripel-Therapie (Wismutsalz plus zwei Antibiotika) und die
2. modifizierte Tripel-Therapie (Säurehemmer plus zwei Antibiotika).

Allerdings sind diese Regime umständliche Eradikationsverfahren mit schlechter Compliance, die bis zu 35 % mit Nebenwirkungen (Bauchschmerzen, Übelkeit, Durchfall, Mundtrockenheit, Geschmacksstörungen und allergische Hautreak-tionen etc.) behaftet sein können. Deshalb wird eine breite Anwendung er-schwert. Ein weiterer großer Nachteil ist die hohe Zahl der täglich einzu-nehmenden Medikamente (12-16 Tabletten/Tag). Dies wird besonders deutlich bei der Quadrupel-Therapie, bei der gleichzeitig zur klassischen Tripel-Therapie ein Säuresekretionshemmer verabreicht wird.

Die in Deutschland propagierte, besser verträgliche Dual-Therapie (Kombination von Amoxicillin mit Omeprazol) ist allerdings nur gering wirksam und scheint bei mit Omeprazol vorbehandelten Patienten und bei Rauchern sogar weitgehend zu versagen.

Bei den Tripel-Therapien werden als antibiotische Komponente in der Regel Amoxicillin, Nitroimidazolverbindungen (Metronidazol, Tinidazol), Tetracyclin sowie neuerdings Makrolide (Clarithromycin) [in 3-4 Teildosen] verabreicht.

Weltweit werden Eradikationsraten von 70 - 90% erreicht. Verschiedene Faktoren können diesen Eradikationserfolg allerdings beeinflussen:
1 An erster Stelle ist die Resistenz des Keimes (Entwicklungsländer: bis zu 60 %, Deutschland: bis zu 10%) gegenüber Metronidazol, dem am häufigsten in der Tripel-Therapie eingesetzten Antibiotikum, zu nennen. Auch bei der Behandlung mit Clarithromycin wird auf den Nachteil einer Resistenzentwicklung von bis zu 10% hingewiesen.
2. Als ein weiterer Faktor ist die oben erwähnte Compliance der Patienten zu nennen.

### Tiermodell

Als ein geeignetes Tiermodell wurde ein H. felis-Mausmodell beschrieben [A. Lee et al., Gastroentrology **99**: 1315-1323 (1990)] und von uns so modifiziert, daß es sich für das Screening und die vergleichende Bewertung o. g. Verbindungen sehr gut eignet.

Das korkenzieherähnliche, ureasebildende Bakterium H. felis ist trotz großer morphologischer Unterschiede mit H. pylori sehr nah verwandt. H. felis ist ein natürlicher Bewohner der Magenmucosa von Hunden und Katzen. Nach oraler Inokulation kolonisieren die Erreger auch den Mäusemagen in ähnlicher Weise wie H. pylori den Magen des Menschen. Die etablierte chronische Langzeitinfektion führt bei keimfreien Mäusen zur aktiven Gastritis und induziert eine entsprechende Immunantwort.

Die im H. felis-Mausmodell ermittelte therapeutische Effektivität von Prüfpräpa-raten wird als sehr prädiktiv für die entsprechende klinische Wirksamkeit angesehen.

Trotz sehr guter In-vitro-Aktivität von Antibiotika (z. B. Amoxicillin oder Erythromycin) gegen H. pylori, zeigen diese nach monotherapeutischer Anwendung klinisch keine signifikante therapeutische Wirkung. Diese Tatsache wird auch duch das H. felis-Mausmodell wiedergegeben. Entsprechend konnte die klinisch anerkannte eradikative Wirkung der klassischen Tripel-Therapie auch im H. felis-Mausmodell bestätigt werden.

Aus der europäischen Patentanmeldung 589 318 (Bayer) sind bereits antibakteriell wirksame 7-(1-Aminomethyl-2-oxa-7-aza-bicyclo[3.3.0]oct-7-yl)-chinolon- und naphthyridoncarbonsäure-Derivate bekanntgeworden. Die überragende Wirkung derartiger Verbindungen zur Bekämpfung von Helicobacter spp. ist bisher unbekannt geblieben. Ferner ist aus der europäischen Patentanmeldung 671 391 (Bayer) bekanntgeworden, daß auch 7-(1-Aminomethyl-2-oxa-7-aza-bicyclo[3.3.0]oct-7-yl)-1-cyclopropyl-6,8-difluor-1,4-dihydro-4-oxo-5-vinyl-3-chinolincarbonsäure gegenüber H. pylori Wirksamkeit besitzt. Diese Wirksamkeit ist aber vergleichsweise gering ausgeprägt. Das ebenfalls in dieser Anmeldung beschriebene 5-Ethinyl-derivat hat sich als instabil erwiesen, weshalb es für eine therapeutische Anwendung nicht in Frage kommt. In der japanischen Patentanmeldung JP 8048629 (Dainippon) wurde beschrieben, daß Verbindungen wie 8-Chlor-1-cyclopropyl-7-([S,S]-2,8-diazabicyclo[4.3.0]non-8-yl)-6-fluor-1,4-dihydro-4-oxo-3-chinolincarbonsäure (BAY Y 3118) eine antibakterielle Wirkung gegenüber H. pylori aufweisen. Es ist auch bekannt, daß eine Reihe von hochwirksamen Chinolonen, wie zum Beipiel Ciprofloxacin, Lomefloxacin oder Ofloxacin (Journal of Antimicrobial Chemotherapy **22**, 631-636 [1988], Antimicrobial Agents and Chemotherapy, **33**, 108-109 [1989]), in vitro gegenüber Helicobacter spp. eine Wirkung aufweisen. Es zeigte sich jedoch im Tiermodell (H. felis, Maus), daß diese klinisch verwendeten antibakteriell wirksamen Chinolone nicht in der Lage sind, zu einer Eradikation des Keimes zu führen. Auch durch eine monotherapeutische Behandlung mit hochwirksamen Chinolonen, die bisher nicht in den Markt eingeführt wurden, wie zum Beispiel mit dem bereits erwähnten BAY Y 3118, kann keine Eradikation von H. felis im Mausmodell erreicht werden. Die Verwendung von Trovafloxacin oder seinen Derivaten in Kombination mit anderen Antibiotika wie Amoxicillin oder Tetracyclinen oder Protonenpumpeninhibitoren wie Omeprazol zur Therapie von H. pylori wird in den Patentanmeldungen EP 676 199 und GB 2 289 674 (Pfizer) beschrieben.

Die EP-A 671 391 betrifft 5-Vinyl- und 5-Ethinyl-chinolon- und -naphthyridon-carbonsäuren; sie wirken antibakteriell und werden als Futterzusatzstoffe empfohlen. U. a. wird auch die Bekämpfung von Helicobacter pylori erwähnt.

Die EP-A 589 318 betrifft Chinolon- und Naphthyridoncarbonsäuren und -carbonsäurederivate, die in 7-Stellung durch einen bicyclischen Rest substituiert sind, der seinerseits an einem der beiden Brückenkopfatome einen Aminomethylrest trägt. Die Druckschrift erwähnt Helicobacter pylori nicht und konnte deswegen die Verwendung bestimmter Verbindungen zu seiner Bekämpfung auch nicht nahelegen.

Die WO 97/31919 ist Stand der Technik im Sinne von Art. 54 (3) EPÜ. Da sich die erfindungsgemäßen Verbindungen von denen der WO 97/31919 durch die Position der Aminoalkylgruppe am 7-ständigen bicyclischen Aminrest unterscheiden, ist die WO 97/31919 nicht neuheitsschädlich.

Es war die Aufgabe der zugrundeliegenden Erfindung, gut verträgliche Wirkstoffe zu finden, die in der Lage sind, dieses hochspezialisierte Bakterium durch eine einfache Monotherapie zu eradizieren.

Es wurde jetzt gefunden, daß die Verbindungen der Formel (I) 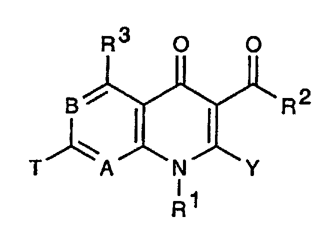 worin
- R¹: für gegebenenfalls durch Halogen oder Hydroxy ein- bis dreifach substituiertes Alkyl mit 1 bis 4 Kohlenstoffatomen, Alkenyl mit 2 bis 4 Kohlenstoffatomen, gegebenenfalls durch 1 oder 2 Fluoratome substituiertes Cycloalkyl mit 3 bis 6 Kohlenstoffatomen, Bicyclo[1.1.1]pent-1-yl, 1,1-Dimethylpropargyl, 3-Oxetanyl, Methoxy, Amino, Methylamino, Dimethylamino, gegebenenfalls durch Halogen, Amino oder Hydroxy ein- oder zweifach substituiertes Phenyl, Isoxazolyl, Thiadiazolyl,
- R²: für Hydroxy, gegebenenfalls durch Hydroxy, Methoxy, Amino, Dimethylamino oder Ethoxycarbonyl substituiertes Alkoxy mit 1 bis 4 Kohlenstoffatomen, Benzyloxy, Allyloxy, Propargyloxy oder (5-Methyl-2-oxo-1,3-dioxol-4-yl)-methyloxy, Acetoxymethyloxy, Pivaloyloxymethyloxy, 5-Indanyloxy, Phthalidinyloxy, 3-Acetoxy-2-oxo-butyloxy, Nitromethyl oder Dialkoxycarbonylmethyl mit 1 bis 2 Kohlenstoffatomen in jedem Alkylteil,
- R³: für Wasserstoff, Amino, Hydroxy, Methyl oder Halogen,
- A: für N oder C-R⁷ steht, worin
R⁷ für Wasserstoff, Halogen, CF₃, OCH₃, OCHF₂, CH₃, CN, CH=CH₂ oder C CH steht oder auch gemeinsam mit R¹ eine Brücke der Struktur -*O-CH₂-CH-CH₃, -*S-CH₂-CH₂-, -*S-CH₂-CH-CH₃, -*CH₂-CH₂-CH-CH₃ oder -*O-CH₂-N-R⁸, wobei das mit * markierte Atom mit dem Kohlenstoffatom von A verknüpft ist und worin R⁸ Wasserstoff, Methyl oder Formyl bedeutet, bilden kann,
- B: für N, C-H, C-F, C-Cl, C-NO₂, C-NH₂,
- Y: für Wasserstoff steht oder gemeinsam mit R² eine Brücke der Struktur -*S-NHbilden kann, wobei das mit * markierte Atom für Y steht, und
- T: einen Rest der Formel 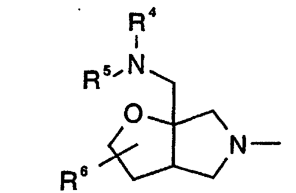 bedeutet, worin
R⁴ für H, CH₃, C₂H₅, gegebenenfalls durch Amino substituiertes Acyl mit 1 bis 5 Kohlenstoffatomen, Alkoxycarbonyl, Aminocarbonyl, Alkylthio-thiocarbonyl und Dialkoxyphosphoryl mit 1 bis 4 Kohlenstoffatomen im Alkylteil,
R⁵ für H, CH₃, C₂H₅ und
R⁶ für H, CH₃ stehen, und deren pharmazeutisch verwendbaren Hydrate und Säureadditionssalze sowie die Alkali-, Erdalkali-, Silber- und Guanidiniumsalze der zugrundeliegenden Carbonsäuren eine hohe antibakterielle Wirkung gegenüber Helicobacter spp. aufweisen und zur Eradikation dieses Erregers verwendet werden können.

Bevorzugt sind die Verbindungen der Formel (I), worin
- R¹: für gegebenenfalls durch Fluor ein- bis dreifach substituiertes Alkyl mit 1 bis 4 Kohlenstoffatomen, Vinyl, gegebenenfalls durch Fluor substituiertes Cyclopropyl, Bicyclo[1.1.1]pent-1-yl, 1,1-Dimethylpropargyl, 3-Oxetanyl, Methylamino, gegebenenfalls durch Fluor, Amino oder Hydroxy ein- oder zweifach substituiertes Phenyl, Thiadiazolyl,
- R²: für Hydroxy, gegebenenfalls durch Ethoxycarbonyl substituiertes Alkoxy mit 1 bis 4 Kohlenstoffatomen, Benzyloxy, Allyloxy, Propargyloxy,
- R³: für Wasserstoff, Amino, Hydroxy, Methyl oder Fluor,
- A: für N oder C-R⁷ steht, worin
R⁷ für Wasserstoff, Halogen, CF₃, OCH₃, OCHF₂, CH₃, CN, CH=CH₂ oder C CH steht oder auch gemeinsam mit R¹ eine Brücke der Struktur -*O-CH₂-CH-CH₃ oder -*O-CH₂-N-R⁸, wobei das mit * markierte Atom mit dem Kohlenstoffatom von A verknüpft ist und worin R⁸ Wasserstoff oder Methyl bedeutet, bilden kann,
- B: für N, C-H, C-F, C-Cl, C-NH₂,
- Y: für Wasserstoff steht oder gemeinsam mit R² eine Brücke der Struktur -*S-NHbilden kann, wobei das mit * markierte Atom für Y steht, und
- T: einen Rest der Formel 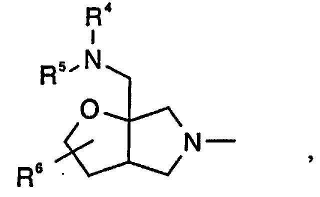 bedeutet, worin
R⁴ für H, CH₃, C₂H₅, gegebenenfalls durch Amino substituiertes Acyl mit 1 bis 5 Kohlenstoffatomen, Alkoxycarbonyl, Aminocarbonyl, Alkylthio-thiocarbonyl und Dialkoxyphosphoryl mit 1 bis 4 Kohlenstoffatomen im Alkylteil,
R⁵ für H, CH₃, C₂H₅ und
R⁶ für H stehen,
und deren pharmazeutisch verwendbaren Hydrate und Säureadditionssalze sowie die Alkali-, Erdalkali-, Silber- und Guanidiniumsalze der zugrundeliegenden Carbonsäuren.

Besonders bevorzugt sind die Verbindungen der Formel (I), worin
- R¹: für gegebenenfalls durch Fluor ein- oder zweifach substituiertes Alkyl mit 1 bis 4 Kohlenstoffatomen, gegebenenfalls durch Fluor substituiertes Cyclopropyl, gegebenenfalls durch Fluor ein- oder zweifach substituiertes Phenyl,
- R²: für Hydroxy, gegebenenfalls durch Ethoxycarbonyl substituiertes Alkoxy mit 1 bis 4 Kohlenstoffatomen, Benzyloxy, Allyloxy, Propargyloxy,
- R³: für Wasserstoff, Amino, Hydroxy, Methyl oder Fluor,
- A: für N oder C-R⁷ steht, worin
R⁷ für Wasserstoff, Chlor, Fluor, OCH₃, OCHF₂, CH₃ oder CN steht oder auch gemeinsam mit R¹ eine Brücke der Struktur -*O-CH₂-CH-CH₃ oder -*O-CH₂-N-CH₃, wobei das mit * markierte Atom mit dem Kohlenstoffatom von A verknüpft ist, bilden kann,
- B: für N, C-H, C-F,
- Y: für Wasserstoff steht oder gemeinsam mit R² eine Brücke der Struktur -*S-NHbilden kann, wobei das mit * markierte Atom für Y steht, und
- **T**: einen Rest der Formel 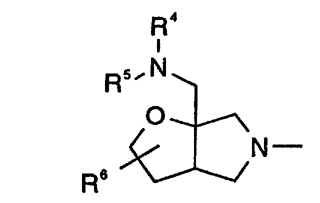 bedeutet, worin
R⁴ für H, CH₃, C₂H₅, gegebenenfalls durch Amino substituiertes Acyl mit 1 bis 4 Kohlenstoffatomen oder Alkoxycarbonyl mit 1 bis 4 Kohlenstoffatomen im Alkylteil,
R⁵ für H und
R⁶ für H stehen,
und deren pharmazeutisch verwendbaren Hydrate und Säureadditionssalze sowie die Alkali-, Erdalkali-, Silber- und Guanidiniumsalze der zugrundeliegenden Carbonsäuren.

Die Verbindungen, die für die erfindungsgemäße Verwendung geeignet sind, sind zum großen Teil bereits aus der europäischen Patentanmeldung 589 318 bekannt oder können nach den dort beschriebenen Verfahren hergestellt werden. So werden die Verbindungen der Formel (I) beispielsweise durch Umsetzung eines bicyclischen Amins T-H mit einem Chinolongrundkörper Q-Hal, wobei Q den am Substituenten T stehenden Quinolonrest in Formel (I) bedeutet und Hal beispielsweise für ein Halogenatom als Abgangsgruppe steht, gemäß der folgenden Reaktionsgleichung erhalten:

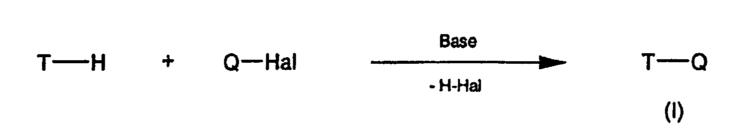

Die zur Herstellung der erfindungsgemäßen Verbindungen der Pomel (I) eingesetzten 7-Halogen-chinoloncarbonsäuredederivate Q-Hal sind bekannt oder können nach bekannten Methoden hergestellt werden. So sind die 7-Chlor-8-cyano-1-cyclopropyl-6-fluor-1,4-dihydro-4-oxo-3-chinolincarbonsäure beziehungsweise der 7-Chlor-8-cyano-1-cyclopropyl-6-fluor-1,4-dihydro-4-oxo-3-chinolincarbonsäure-ethylester in der europäischen Patentanmeldung 276 700 beschrieben worden. Die entsprechenden 7-Fluorderivate lassen sich beispielsweise auch über die folgende Reaktionsfolge aufbauen:
- A: für N oder C-R⁷ steht, worin
R⁷ für Wasserstoff, Chlor, Fluor, OCH₃, OCHF₂, CH₃ oder CN steht oder auch gemeinsam mit R¹ eine Brücke der Struktur -*O-CH₂-CH-CH₃ oder -*O-CH₂-N-CH₃, wobei das mit * markierte Atom mit dem Kohlenstoffatom von A verknüpft ist, bilden kann,
- B: für N, C-H, C-F,
- Y: für Wasserstoff steht oder gemeinsam mit R² eine Brücke der Struktur -*S-NH- bilden kann, wobei das mit * markierte Atom für Y steht, und

- T: einen Rest der Formel 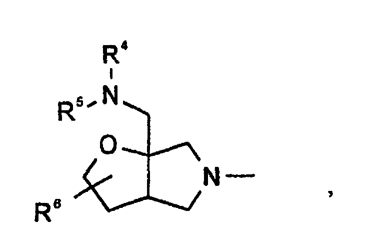
worin
- R⁴: für H, CH₃, C₂H₅, gegebenenfalls durch Amino substituiertes Acyl mit 1 bis 4 Kohlenstoffatomen oder Alkoxycarbonyl mit 1 bis 4 Kohlenstoffatomen im Alkylteil,
- R⁵: für H und
- R⁶: für H steht, bedeutet,
und deren pharmazeutisch verwendbaren Hydrate und Säureadditionssalze sowie die Alkali-, Erdalkali-, Silber- und Guanidiniumsalze der zugrundeliegenden Carbonsäuren.

Die Verbindungen, die für die erfindungsgemäße Verwendung geeignet sind, sind zum großen Teil bereits aus der europäischen Patentanmeldung 589 318 bekannt oder können nach den dort beschriebenen Verfahren hergestellt werden. So werden die Verbindungen der Formel (I) beispielsweise durch Umsetzung eines bicyclischen Amins T-H mit einem Chinolongrundkörper Q-Hal, wobei Hal beispielsweise für ein Halogenatom als Abgangsgruppe steht, gemäß der folgenden Reaktionsgleichung erhalten:

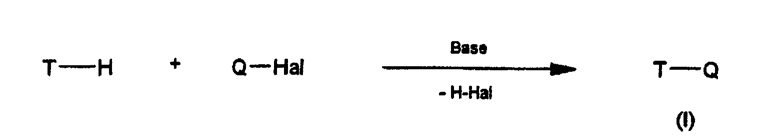

Die zur Herstellung der erfindungsgemäßen Verbindungen der Fomel (I) eingesetzten 7-Halogen-chinoloncarbonsäuredederivate Q-Hal sind bekannt oder können nach bekannten Methoden hergestellt werden. So sind die 7-Chlor-8-cyano-1-cyclopropyl-6-fluor-1,4-dihydro-4-oxo-3-chinolincarbonsäure beziehungsweise der 7-Chlor-8-cyano-1-cyclopropyl-6-fluor-1,4-dihydro-4-oxo-3-chinolincarbonsäure-ethylester in der europäischen Patentanmeldung 276 700 beschrieben worden. Die entsprechenden 7-Fluorderivate lassen sich beispielsweise auch über die folgende Reaktionsfolge aufbauen:

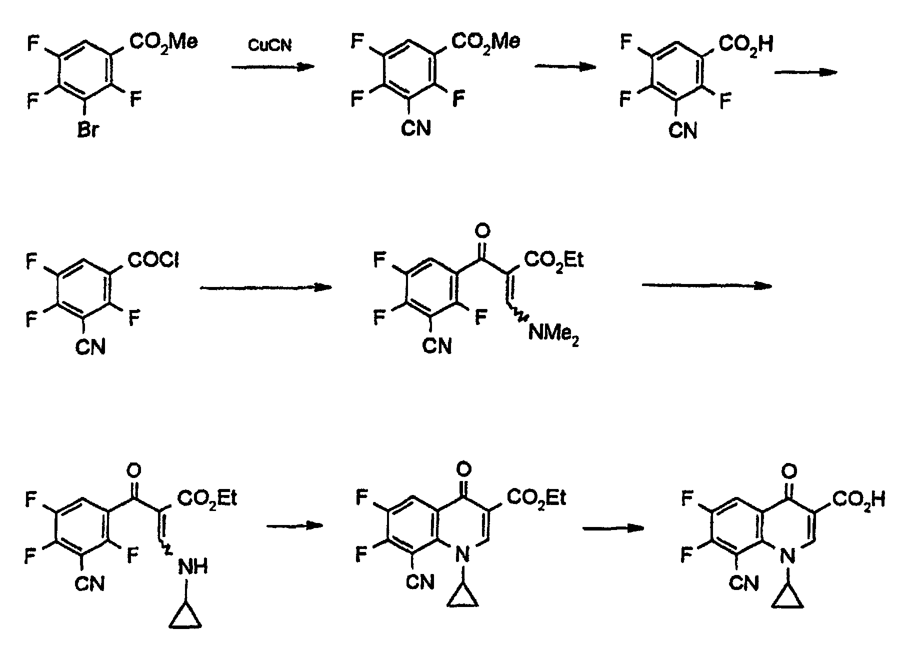

Ein Alternatiwerfahren zur Herstellung der Zwischenverbindung 2,4-Dichlor-3-cyano-5-fluor-benzoylchlorid (europäische Patentanmeldung 276 700), das in 3-Cyano-2,4,5-trifluor-benzoylfluorid überführt werden kann, geht von 5-Fluor-1,3-xylol aus: 5-Fluor-1,3-xylol wird in Gegenwart eines Katalysators unter ionischen Bedingungen im Kern zweifach zum 2,4-Dichlor-5-fluor-1,3-dimethylbenzol chloriert und dieses anschließend unter radikalischen Bedingungen in den Seitenketten zu 2,4-Dichlor-5-fluor-3-dicblormethyl-1-trichlormethylbenzol chloriert. Dieses wird über die 2,4-Dichlor-5-fluor-3-dichlormethylbenzoesäure zur 2,4-Dichlor-5-fluor-3-formylbenzoesäure verseift und anschließend zur 2,4-Dichlor-5-fluor-3-*N*-hydroxyiminomethyl-benzoesäure umgesetzt. Durch Behandlung mit Thionylchlorid wird 2,4-Dichlor-3-cyano-5-fluor-benzoylchlorid erhalten, welches noch durch einen Chlor/Fluor-Austausch zu 3-Cyano-2,4,5-trifluor-benzoylfluorid umgesetzt werden kann.

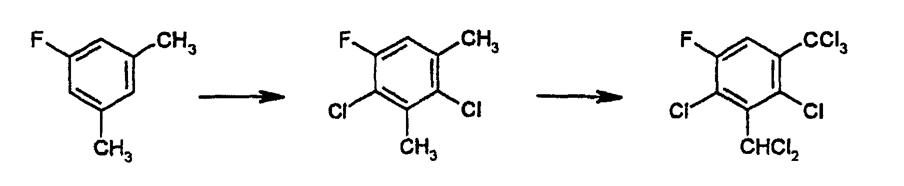

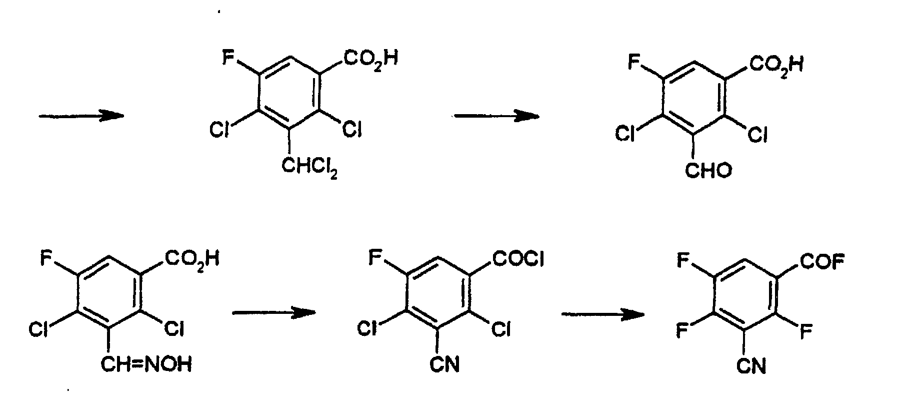

In den eingesetzten bicyclischen Basen T-H sind die beiden Fünfringe cis-verknüpft. Die Basen T-H können als Racemate oder als enantiomerenreine Verbindungen eingesetzt werden. Zur Herstellung der enantiomerenreinen Amine T-H kommen verschiedene Verfahren in Frage:
1. Die racemischen bicyclischen Amine (T-H) können mit enantiomerenreinen Säuren, zum Beispiel Carbonsäuren oder Sulfonsäuren wie N-Acetyl-L-glutaminsäure, N-Benzoyl-L-alanin, 3-Brom-campher-9-sulfonsäure, Campher-3-carbonsäure, cis-Camphersäure, Campher-10-sulfonsäure, O,O'-Dibenzoylweinsäure, D- oder L-Weinsäure, Mandelsäure, -Methoxy-phenylessigsäure, 1-Phenyl-ethansulfonsäure, -Phenyl-bernsteinsäure zu einem Gemisch der diastereomeren Salze umgesetzt werden, die sich durch fraktionierte Kristallisation zu den diastereomerenreinen Salzen trennen lassen. Durch Behandlung dieser Salze mit Alkali- oder Erdalkalihydroxyden lassen sich die enantiomerenreinen Amine freisetzen.
2. In ähnlicher Weise wie unter 1. beschrieben läßt sich eine Racematspaltung der basischen Zwischenstufen, die bei der Herstellung der racemischen bicyclischen Amine auftreten, mit den oben aufgeführten enantiomerenreinen Säuren durchführen.
3. Sowohl die racemischen Amine (T-H) als auch einige der zu den bicyclischen Aminen (T-H) führenden Zwischenstufen können, gegebenenfalls nach Acylierung, an chiralen Trägermaterialien chromatographisch getrennt werden.
4. Die racemischen Amine (T-H) können auch durch chemische Verknüpfung mit chiralen Acylresten in Diastereomerengemische überführt werden, die sich durch Destillation, Kristallisation oder Chromatographie in die diastereomerenreinen Acylderivate trennen lassen, aus denen sich durch Verseifung die enantiomerenreinen Amine isolieren lassen. Beispiele für Reagentien zur Verknüpfung mit chiralen Acylresten sind: -Methoxy- -trifluormethyl-phenylacetylchlorid, Menthylisocyanat, D- oder L- -Phenyl-ethyl-isocyanat, Chlorameisensäure-menthylester, Campher-10-sulfonsäurechlorid.
5. Im Verlauf der Synthese der bicyclischen Amine (T-H) können statt achiraler auch chirale Schutzgruppen eingeführt werden. Man gelangt auf diese Weise zu Diastereomerengemischen, die sich auftrennen lassen. Zum Beispiel kann in der Zwischenstufe 7-Benzyl-2-oxa-7-azabicyclo[3.3.0]octan-1-carbonitril der Benzylrest durch einen -Phenylethylrest in der R- oder S-Konfiguration ersetzt sein.

Als Beispiele für erfindungsgemäße Verbindungen seien außer den in den Herstellungsbeispielen aufgeführten Verbindungen die in folgender Tabelle 1 aufgeführten Verbindungen genannt, die sowohl in racemischer Form als auch als enantiomerenreine beziehungsweise diastereomerenreine Verbindungen verwendet werden können.

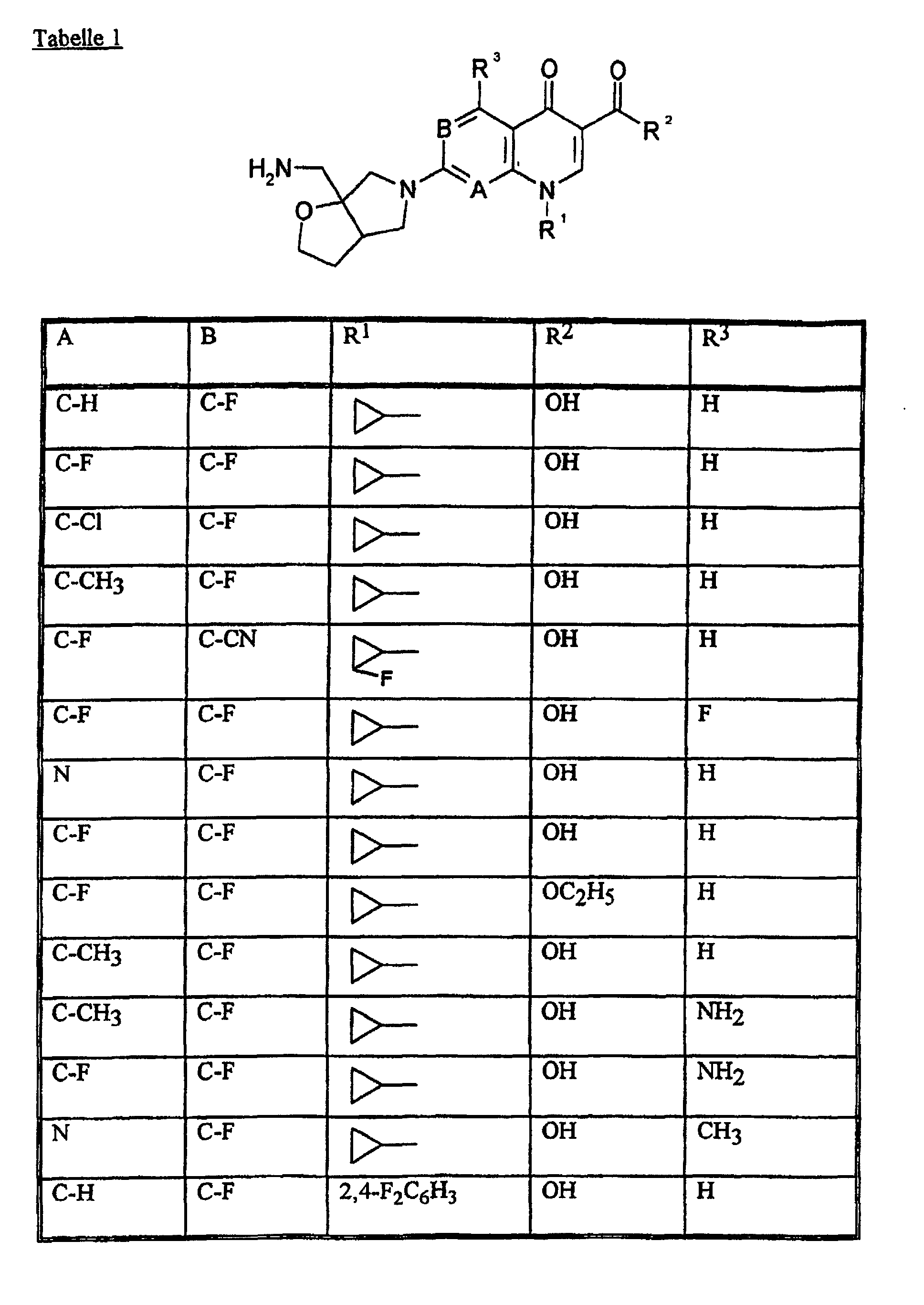

Die erfindungsgemäßen Verbindungen wirken stark antibiotisch und zeigen bei geringer Toxizität ein breites antibakterielles Spektrum gegenüber grampositiven und gramnegativen Keimen, vor allem aber auch gegenüber Helicobacter spp.

Diese wertvollen Eigenschaften ermöglichen ihre Verwendung als chemotherapeutische Wirkstoffe zur Therapie von Helicobäcter-pylori-Infektionen und den damit assoziierten gastroduodenalen Erkrankungen, die durch die erfindungsgemäßen Verbindungen verhindert, gebessert und/oder geheilt werden können.

Die erfindungsgemäßen Verbindungen können in verschiedenen pharmazeutischen Zubereitungen angewendet werden. Als bevorzugte pharmazeutische Zubereitungen seien Tabletten, Dragees, Kapseln, Pillen, Granulate, Lösungen, Suspensionen und Emulsionen genannt.

Obwohl die erfindungsgemäßen Verbindungen als monotherapeutische Mittel eingesetzt werden, können sie bei Bedarf auch in Kombination mit anderen Therapeutika verwendet werden. Beispielhaft seien als Kombinationspartner genannt: Nitroimidazolderivate, zum Beispiel Metronidazol; Protonenpumpeninhibitoren, zum Beispiel Omeprazol, Pantoprazol oder Lanzoprazol; H₂-Rezeptorantagonisten, wie zum Beispiel Cimetidin, Ranitidin, Famotidin oder Nizatidin; Wismutverbindungen, wie zum Beispiel Wismutsalicylat oder CBS (colloidales Bismut Subcitrat); andere Antibiotika, wie zum Beispiel Amoxicillin, Azlocillin oder Clarithromycin; Antacida.

In der folgenden Tabelle 2 sind die minimalen Hemmkonzentrationen einiger erfindungsgemäßer Verbindungen im Vergleich zu der strukturell ähnlichen 7-(1-Aminomethyl-2-oxa-7-aza-bicyclo[3.3.0]oct-7-yl)-1-cyclopropyl-6,8-difluor-1,4-dihydro-4-oxo-5-vinyl-3-chinolincarbonsäure (europäische Patentanmeldung 671 391) und zu Ciprofloxacin beispielhaft als Maß für die antibakterielle Wirksamkeit aufgeführt.

Die minimalen Hemmkonzentrationen (MHK) wurden im Agar-Verdünnungstest auf Columbia-Agar beziehungsweise Basis 2 Agar (Oxoid) mit 10 % lysiertem Pferdeblut entweder bei pH 7 oder pH 5 mit 1 g/l Harnstoff bestimmt. Die Prüfsubstanzen wurden in Replica-Schalen getestet, die bei jeweils doppelter Verdünnung abfallende Konzentrationen des Wirkstoffes enthielten. Zum Beimpfen wurden frische Helicobacter-Kulturen aus Flüssigkultur oder Suspension der Keime von Agarplatten verwandt. Die beimpften Agarplatten wurden bei 37°C in einer Atmosphäre mit 5-10 % CO₂ während 48-72 Stunden bebrütet. Der abgelesene MHK-Wert (µg/ml) gibt die geringste Wirkstoffkonzentration an, bei der mit bloßem Auge kein Wachstum zu erkennen war. Die folgenden Helicobacter-Isolate wurden verwendet: H. felis ATCC 49179, H. pylori NCTC 11637, H. pylori-Klinikisolat 008.

**Tabelle 2:**

| MHK-Werte (mg/l) einiger erfindungsgemäßer Verbindungen im Vergleich zur Referenzverbindung (Ref.^{*)}) (Agarverdünnungstest) | | |
|---|---|---|
| Beispiel | MHK(mg/l) | |
| | H. pylori 008 | H. pylori 11637 |
| 1 B | 0,06 | 0,25 |
| 1 BA | 0,06 | 0,06 |
| 2 B | 0,06 | 0,5 |
| 5 B | 0,03 | 0,03 |
| 5 C | 0,03 | 0,03 |
| 5D | 1 | 1 |
| 6 C | 0,03 | 0,03 |
| 9 B | <2 | <2 |
| 10 B | 0,06 | 0,06 |
| 11 B | ≤0,015 | 0,03 |
| 12 B | ≤0,015 | 0,03 |
| 12 C | ≤0,015 | 0,03 |
| 13 B | ≤0,015 | 0,03 |
| 14 B | ≤0,015 | 0,125 |
| 14 C | ≤0,015 | 0,03 |
| 16 B | 0,25 | 0,125 |
| 17 B | 0,125 | 0,25 |
| 18 B | 0,125 | 0,125 |
| 23 B | ≤0,015 | ≤0,015 |
| 24 B | 0,06 | 0,125 |
| 26 B | 0,06 | 0,25 |
| 27 | ≤0,015 | 0,5 |
| 29 | 8 | 8 |
| 30 | 0,25 | 0,125 |
| 31 | <0,5 | 0,5 |
| 34 B | 0,125 | 0,25 |
| Ciprofloxacin | 0,125 | 0,125 |
| Ref.^{*)} | 0,25 | 0,25 |

| | | |
|---|---|---|
| ^{*)} Ref. = 7-(1-Aminomethyl-2-oxa-7-aza-bicyclo[3.3.0]oct-7-yl)-1-cyclopropyl-6,8-difluor-1,4-dihydro-4-oxo-5-vinyl-3-chinolincarbonsäure-Hydrochlorid (europäische Patentanmeldung 671 391) | | |

In Tabelle 3 ist als Beispiel für die überraschend hohe In-vivo-Wirkung der erfindungsgemäßen Verbindungen der Therapieerfolg nach 3-tägiger Behandlung infizierter Mäuse mit rac. 7-(1-Aminomethyl-2-oxa-7-aza-bicyclo[3.3.0]oct-7-yl)-1-cyclopropyl-6,8-difluor-1,4-dihydro-4-oxo-3-chinolincarbonsäure-Hydrochlorid sowie für 1S,5R(+)-7-(1-Aminomethyl-2-oxa-7-aza-bicyclo[3.30]oct-7-yl)-8-cyano-1-cyclopropyl-6-fluor-1,4-dihydro-4-oxo-3-chinolincarbonsäure-Hydrochlorid (Beispiel 5 C) im Vergleich zur Behandlung mit Ciprofloxacin aufgeführt: Während mit Ciprofloxacin nur eine Clearancerate von 17 % erreicht wird, beträgt diese bei den erfindungsgemäßen Verbindungen 67 % beziehungsweise 100 %. Eine 14-tägige Behandlung der Mäuse mit 3 x 10 mg/kg rac. 7-(1-Aminomethyl-2-oxa-7-aza-bicyclo[3.3.0]oct-7-yl)-1-cyclopropyl-6,8-difluor-1,4-dihydro-4-oxo-3-chinolincarbonsäure-Hydrochlorid führte sogar zu einer totalen Eradikation des Keimes.

Für Untersuchungen im Tiermodell wurden weibliche Swiss Mäuse (8 bis 12 Wochen alt, SPF-Zucht) mit handelsüblichem Futter und Wasser gehalten. Zur Kolonisation wurde ein definierter H.-felis-Stamm (ATCC 49179) verwendet. Die Bakterien werden als Suspension (0,1 ml mit 10⁸-10⁹ Bakterien) 4-mal innerhalb von 7 Tagen per Schlundsonde appliziert. Alternativ hierzu wurden zur Infektion auch Magenhomogenate von vorher infizierten Mäusen verwendet.

3-5 Tage nach Etablierung der Infektion wurde die Behandlung mit Prüfpräparaten begonnen. Als erster Behandlungserfolg wurde die Keimreduktion als "Clearance" 24 Stunden nach der letzten Behandlung (zum Beispiel 3, 7, 10, 14 Tage; 1-3-mal täglich) bestimmt. In einigen Fällen wurde auch die Keim-Eradikation 2-4 Wochen nach Behandlungsende ermittelt. In Anlehnung an den in der klinischen Diagnostik verwendeten "CLO"-Test wurde ein Urease-Test auf Mikrotiter-Basis eingesetzt. Geprüft wurden definierte Magenbiopsate auf Farbumschlag innerhalb 24 Stunden.

**Tabelle 3:**

| Therapieerfolg nach 3-tägiger Behandlung von infizierten Mäusen (6 Tiere pro Gruppe) | | | |
|---|---|---|---|
| Präparat | Dosis [mg/kg] | Clearance | % |
| Substanz A | 2x10 | 4/6 | 67 |
| Substanz B | 2x10 | 6/6 | 100 |
| Ciprofloxacin | 2x10 | 1/6 | 17 |

### Beispiele

### Herstellung der Zwischenprodukte

### Beispiel Z 1

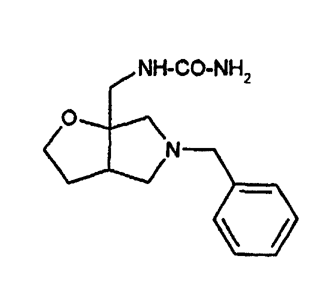

4,64 g (20 mmol) 1-Aminomethyl-7-benzyl-2-oxa-7-aza-bicyclo[3.3.0]octan werden in 20 ml Wasser mit 20 ml ln-HCl bei 25°C in Lösung gebracht. Man fügt eine Lösung von 1,98 g (22 mmol) Kaliumcyanat in 20 ml Wasser zu und erhitzt 1 Stunde unter Rückfluß. Die Mischung wird bei 60°C/15 mbar eingeengt, der Rückstand mit Dichlormethan eluiert, das Eluat mit Natriumsulfat getrocknet und eingeengt. Man erhält ein Öl (7,3 g), das mit Dichlormethan/Methanol/17 % Ammoniak (150:20:1) an Kieselgel chromatographisch gereinigt wird.

Ausbeute: 4,0 g (72 % der Theorie) 7-Benzyl-1-ureidomethyl-2-oxa-7-aza-bicyclo[3.3.0]octan als Öl,
¹H-NMR (400 MHz; CDCl₃): 4,9 breit (NH₂), 5,7 ppm breit (NH).

### Beispiel Z 2

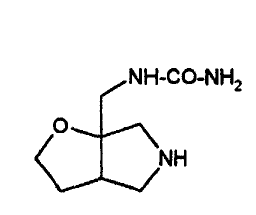

3,9 g (14 mmol) 7-Benzyl-1-ureidomethyl-2-oxa-7-aza-bicyclo[3.3.0]octan werden in 70 ml Ethanol in Gegenwart von 2 g Pd-C bei 100°C/100 bar hydriert, der Kontakt abfiltriert, die Lösung eingeengt und das erhaltene Produkt (2,8 g zähes Öl) chromatographisch gereinigt.

Ausbeute: 1,5 g (58 % der Theorie) 1-Ureidomethyl-2-oxa-7-aza-bicyclo[3.3.0]octan als zähes Öl,
FAB-MS: m/e 186 ([(M+H)⁺], 371 [(2M+H)⁺].

### Beispiel Z 3

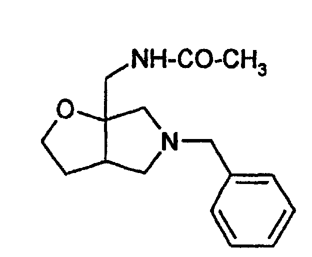

7 g (30 mmol) 1-Aminomethyl-7-benzyl-2-oxa-7-aza-bicyclo[3.3.0]octan werden in 50 ml Dioxan vorgelegt, mit 3 g (30 mmol) Triethylamin versetzt und unter Eiskühlung innerhalb 10 Minuten 3 g (30 mmol) Essigsäureanhydrid zugetropft. Man rührt über Nacht bei Raumtemperatur nach und engt bei 60°C/20 mbar ein. Der erhaltene Rückstand wird an Kieselgel mit Dichlormethan/Methanol/17 %-Ammoniak (150/20/1) als Laufmittel chromatographisch gereinigt.

Ausbeute: 8,2 g 1-Acetylaminomethyl-7-benzyl-2-oxa-7-aza-bicyclo[3.3.0]octan als Öl; Gehalt (nach GC): 94 %ig.
Massenspektrum: 274 (M⁺), 202, 184, 170, 91 (100%).

### Beispiel Z 4

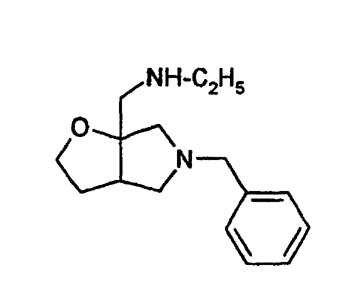

7,1 g (25,9 mmol) 1-Acetylaminomethyl-7-benzyl-2-oxa-7-aza-bicyclo[3.3.0]octan werden in 50 ml Tetrahydrofuran gelöst und diese Lösung zu einer Suspension von 3,1 g (82 mmol) Lithiumaluminiumhydrid in 60 ml Tetrahydrofuran so zugetropft, daß die Temperatur auf 31° bis 33°C gehalten wird. Anschließend erhitzt man 12 Stunden unter Rückfluß und zersetzt unter Kühlung mit 1,2 ml Wasser und 1,2 ml 15 %-iger Natronlauge. Der ausgefallene Niederschlag wird abgesaugt, mit Tetrahydrofuran gewaschen und das Filtrat eingeengt. Das zurückbleibende Öl (5,5 g) wird an Kieselgel chromatographisch gereinigt (Laufmittel: Dichlormethan/Methanol/17 %-iges Ammoniak 150:20:1).

Ausbeute: 4,55 g (67,5 % der Theorie) 7-Benzyl-1-ethylaminomethyl-2-oxa-7-azabicyclo[3.3.0]octan als Öl.

### Beispiel Z 5

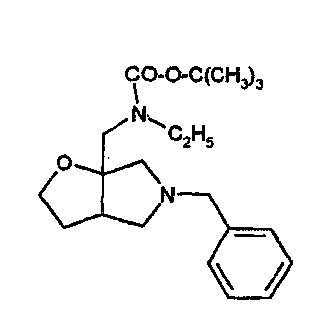

1,4 g (5,4 mmol) 7-Benzyl-1-ethylaminomethyl-2-oxa-7-aza-bicyclo[3.3.0]octan werden in 6 ml tert.-Butanol gelöst und mit einer Lösung von 220 mg (5,5 mmol) NaOH in 4 ml Wasser und 1,24 g (5,7 mmol) Di-tert.-butyl-dicarbonat versetzt. Die Temperatur steigt auf 27°-31°C an. Man rührt über Nacht nach, engt die Mischung ein, verrührt den Rückstand mit ca. 20 ml Wasser und extrahiert mit Dichlormethan. Der Extrakt wird mit Natriumsulfat getrocknet und im Vakuum eingeengt.

Ausbeute: 1,58 g (81,5 % der Theorie) 7-Benzyl-1-(N-tert.-butoxycarbonyl-N-ethylaminomethyl)-2-oxa-7-aza-bicyclo[3.3.0]octan als Öl.

### Beispiel Z 6

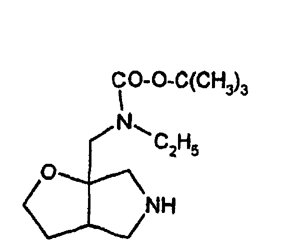

3,5 g (9,7 mmol) 7-Benzyl-1-(N-tert.-butoxycarbonyl-N-ethylaminomethyl)-2-oxa-7-aza-bicyclo[3.3.0]octan werden in 60 ml Ethanol in Gegenwart von 0,8 g Pd-C (10 %) bei 70°C/90 bar hydriert. Der Katalysator wird abfiltriert, mit Ethanol gewaschen und das Filtrat eingeengt. Der Rückstand wird chromatographisch an Kieselgel (Laufmittel: Dichlormethan/Methanol 95:5) gereinigt.

Ausbeute: 2,0 g (76 % der Theorie) 1-(N-tert.-Butoxycarbonyl-N-ethylaminomethyl)-2-oxa-7-aza-bicyclo[3.3,0]octan als Öl.

### Beispiel Z 7

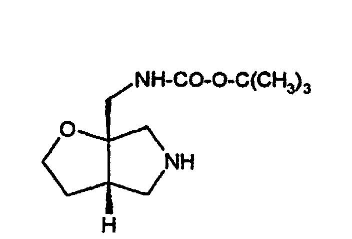

A: 7-Benzyl-1-cyano-2-oxa-7-azabicyclo[3.3.0]octan (5-Benzyl-hexahydro-furo-[2,3-c]pyrrol-6a-carbonitril) wird nach der Vorschrift der europäischen Patentanmeldung 589 318 hergestellt und chromatographisch (Kieselgel, Dichlormethan) gereinigt. Diese racemische Verbindungung wurde über eine chromatographische Racematspaltung (Trägermaterial: Daicel-Chiracell OJ) in die Enantiomeren aufgetrennt, die über die nachfolgenden Schritte entsprechend den Vorschriften der europäischen Patentanmeldung 589 318 weiter umgesetzt wurden.
B: 1,67 g (7,3 mmol) (-)-7-Benzyl-1-cyano-2-oxa-7-azabicyclo[3.3.0]octan werden in 23 ml absolutem Tetrahydrofuran mit 362 mg Lithiumaluminiumhydrid während 15 Stunden unter Rückfluß reduziert. Die Suspension wird mit wäßriger Kalilauge versetzt, anorganische Salze abgesaugt, das Filtrat eingeengt und chromatographisch gereinigt (Kieselgel, Dichlormethan/Methanol 2:1).
   Ausbeute: 1,30 g (76,6 % der Theorie) (-)-1-Aminomethyl-7-benzyl-2-oxa-7-azabicyclo[3.3.0]octan als Öl,
   [α]⁰ : -6,9° (c = 1,3, Dichlormethan).
C: 1,2 g (5,2 mmol) (-)-1-Aminomethyl-7-benzyl-2-oxa-7-azabicyclo[3.3.0]octan werden in 6,5 ml tert.-Butanol mit 254 mg NaOH in 5,1 ml Wasser versetzt 1,3 g Pyrokohlensäure-di-tert.-butylester zugegeben und bei Raumtemperatur ca. 30 Minuten gerührt. Man extrahiert mit Dichlormethan, trocknet Kaliumcarbonat und engt ein. Das erhaltene Rohprodukt wird wird chromatographisch gereinigt (Kieselgel/Dichlormethan 10:1).
   Ausbeute: 1,69 g (98 % der Theorie) (-)-(1S,5S)-1-tert.-Butoxycarbonylaminomethyl-7-benzyl-2-oxa-7-aza-bicyclo[3.3.0]octan als Öl,
   [α]⁰ : -11,8° (c = 1,16, Dichlormethan).
D: 1,56 g (4,49 mmol) (-)-(1S,5S)-1-tert.-Butoxycarbonylaminomethyl-7-benzyl-2-oxa-7-aza-bicyclo[3.3.0]octan werden in 50 ml Ethanol in Gegenwart von 0,5 g Pd-C (10 %) bei 100°C/90 bar hydriert. Man filtriert, engt ein und chromatographiert den Rückstand mit Dichlormethan/Methanol/17 %-Ammoniak (150:20:1) als Laufmittel über Kieselgel. Das eingeengte Eluat kristallisiert durch.

Ausbeute: 790 mg (73 % der Theorie) (-)-(1S,5S)-1-tert.-Butoxycarbonylaminomethyl-2-oxa-7-aza-bicyclo[3.3.0]octan,
Schmelzpunkt: 104-105°C,
[α]⁶ : -10,2° (0,56, CHCl₃).

### Beispiel Z 8

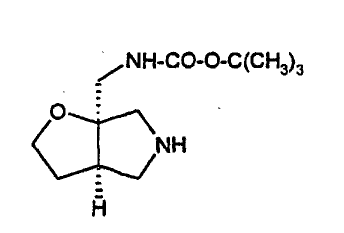

5,6 g (16,8 mmol) (+)-(1R,5R)-1-tert.-Butoxycarbonylaminomethyl-7-benzyl-2-oxa-7-aza-bicyclo[3.3.0]octan, [ ]⁰ : +13,1° (c = 1,1, Dichlormethan, das analog den Vorschriften in Beispiel Z 7 A bis C hergestellt wird, werden in 100 ml Ethanol in Gegenwart von 1 g Pd-C (10 %) bei 70°C/20 bar hydriert. Man filtriert, engt ein und chromatographiert den Rückstand (3,5 g) mit Dichlormethan/Methanol (95:5) als Laufmittel über Kieselgel. Das eingeengte Eluat kristallisiert durch.

Ausbeute: 2,54 g (63 % der Theorie) (+)-(1R,5R)-1-tert.-Butoxycarbonylaminomethyl-2-oxa-7-aza-bicyclo[3.3.0]octan,
Schmelzpunkt: 102-103°C,
[α]⁶ : +7,0° (0,3, CHCl₃).

### Beispiel Z 9A

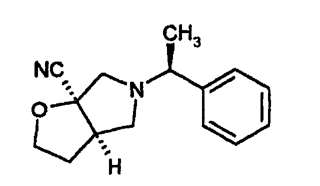

Eine Lösung von 83 g (0,87 mol) 2-Cyano-4,5-dihydrofuran in 2000 ml absolutem Ethylacetat wird bei 15 C vorgelegt. Dazu werden bei dieser Temperatur zuerst 0,5 ml Trifluoressigsäure und nachfolgend 227 g (0,77 mol, 85%ig) N-Methoxymethyl-N-[(S)-1-phenylethyl]-N-trimethylsilylmethylamin innerhalb von 10 Minuten zugetropft. Dabei steigt die Temperatur auf 45 C. Der Reaktionsansatz wird über Nacht bei Raumtemperatur gerührt und anschließend mit 200 ml gesättigter Natriumhydrogencarbonatlösung gewaschen. Die organische Phase wird über Natriumsulfat getrocknet und im Vakuum eingeengt. Das Rohprodukt wird säulenchromatographisch über Kieselgel (Toluol/Ethylacetat, 99:1) gereinigt, wobei das Diastereomerengemisch erhalten wird (56 g, Verhältnis der Diastereomeren A : B = 1,6 : 1). Zur Kristallisation wird der Rückstand in 100 ml Diethylether gelöst und langsam auf - 35 C gekühlt. Die ausgefallenen Kristalle werden über eine gekühlte Fritte abfiltriert und mit wenig auf -60 C gekühltem Diethylether nachgewaschen.

Ausbeute: 13,3 g (7,1% der Theorie) (1R,5R)-1-Cyano-7-[(S)-1-phenylethyl]-2-oxa-7-azabicyclo[3.3.0]octan (Diastereomer A),
Schmelzpunkt: 68°C,
[α]⁵ : -34° (c = 0,99, Methanol). Die Bestimmung der Absolutkonfiguration erfolgte durch eine Röntgenstrukturanalyse.

### Beispiel Z 9B

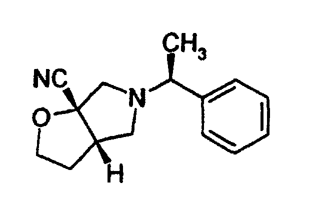

Das Diastereomer B wird durch selektive Kristallisation als Maleinsäure-Salz aus dem Gemisch gewonnen, aus dem vorher Diastereomer A erhalten wurde. Beispielhaft werden zu einer Lösung von der Mutterlauge aus Beispiel Z 9A (103,3 g, 0,427 mol; Diastereomerenverhältnis: 31 : 69, A : B) in 21 Ethylacetat 49,5 g (0,427 mol) Maleinsäure gegeben. Das Gemisch wird 2 Stunden unter Rückfluß erhitzt und über Nacht auf Raumtemperatur abgekühlt. Anschließend werden die Kristalle abgesaugt, in Ethylaceteat und 1N Natronlauge geschüttelt, die organische Phase wird getrocknet und eingeengt. Das Diastereomer B wird so in einer Reinheit von >96% ee erhalten.

Ausbeute: 32 g (45% der Theorie) (1S,5S)-1-Cyano-7-[(S)-1-phenylethyl]-2-oxa-7-azabicyclo[3.3.0]octan (Diastereomer B).

Die mit Maleinsäure-Salz des Diastereomers A angereicherte Ethylacetatlösung wird mehrmals mit Wasser extrahiert, die wäßrige Phase auf pH 8 gestellt und mit Ethylacetat extrahiert. Die organische Phase wird anschließend getrocknet und eingeengt, wobei ein mit Diastereomer A angereichertes Isomerengemisch erhalten wird. Mit diesem kann nun die selektive Kristallisation des Diastereomers A aus Ethylacetat, wie in Beispiel Z 9A beschrieben, durchge
führt werden.
Auf diese Weise kann das Diastereomerengemisch in mehreren Schritte vollständig getrennt werden, so daß sich die Ausbeute von Beispiel Z 9A auf ca. 16% der Theorie beziehungsweise. 26% bezogen auf den Anteil an Diastereomer A erhöhen läßt.

### Beispiel 10A

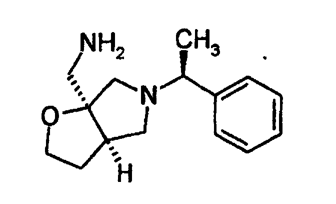

Zu 3,15 g (0,083 mol) Lithiumaluminiumhydrid in 70 ml absolutem Tetrahydrofuran wird bei 30 C eine Lösung aus 16,1 g (0,067 mol) (1R,5R)-1-Cyano-7-[(S)-1-phenylethyl]-2-oxa-7-azabicyclo[3.3.0]octan in 70 ml absolutem Tetrahydrofuran getropft. Der Reaktionsansatz wird dann 15 Stunden unter Rückfluß erhitzt, anschließend auf 10°C gekühlt und nacheinander mit 3,5 ml Wasser, 3,5 ml 15%-iger Kaliumhydroxidlösung und 3,5 ml Wasser versetzt. Der Niederschlag wird abfiltriert, mit Tetrahydrofuran gewaschen und das Filtrat anschließend im Vakuum eingeengt.

Ausbeute: 15,9 g (1S,5R)-1-Aminomethyl-7-[(S)-1-phenylethyl]-2-oxa-7-azabicyclo[3.3.0]octan als Rohprodukt,
[α]⁵ : -38,0 (c = 1,21, Methanol).

### Beispiel Z 10B

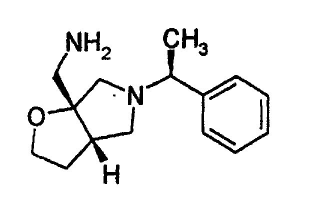

Eine Lösung von 15 g (0,062 mol) (1R,5S)-1-Cyano-7-[(S)-l-phenylethyl] -2-oxa-7-azabicyclo[3.3.0]octan in 30 ml Tetrahydrofuran wird nach Zugabe von 50 ml flüssigem Ammoniak und 5 g Raney-Nickel 5 Stunden bei 80°C unter einem WasserstoffDruck von 110 bis 120 bar hydriert. Nach vollständigem Umsatz wird der Ansatz über Celite filtriert und eingeengt.

Ausbeute: 12,5 g (82% der Theorie) (1R,5S)-1-Aminomethyl-7-[(S)-1-phenylethyl]-2-oxa-7-azabicyclo[3.3.0]octan

### Beispiel Z 11

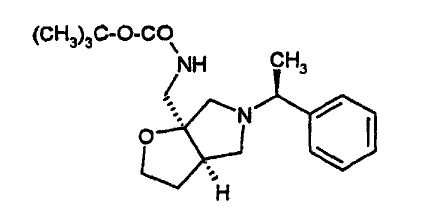

Zu einer Lösung aus 13,4 g (0,545 mol) (1S,5R)-1-Aminomethyl-7-[(S)-1-phenylethyl]-2-oxa-7-azabicyclo[3.3.0]octan in 60 ml Dioxan werden bei 10 C nacheinander 6,4 g (0,6 mol) Natriumcarbonat und 13,0 g (0,6 mol) Di-tert.-butyldicarbonat gegeben. Der Ansatz wird eine Stunde bei Raumtemperatur gerührt und anschließend im Vakuum eingeengt. Der Rückstand wird säulenchromatographisch über Kieselgel (Dichlormethan/Methanol, 95 : 5) gereinigt.

Ausbeute: 18,9 g (quantitativ) (1S,5R)-1-tert.-Butoxycarbonylaminomethyl-7-[(S)-1-phenylethyl]-2-oxa-7-azabicyclo[3.3.0]octan,
[α]⁵ : - 19,0 (c= 1,5 in Methanol).

### Beispiel Z 12

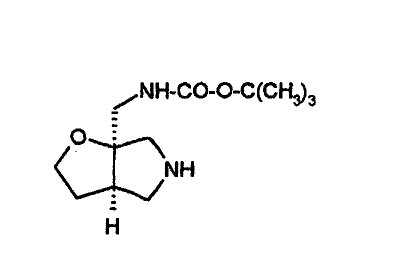

18,0 g (0,052 mol) (1S,5R)-1-tert.-Butoxycarbonylaminomethyl-7-[(S)-1-phenylethyl]-2-oxa-7-azabicyclo[3.3.0]octan werden, in 150 ml absolutem Methanol gelöst, nach Zugabe von 2,5 g 10% Palladium-Aktivkohle 9 h bei 70 C unter 20 bar hydriert. Der Katalysator wird abfiltriert, und das Filtrat im Vakuum eingeengt.

Ausbeute: 12,5 g (99% der Theorie) (1R,5R)-1-tert.-Butoxycarbonylaminomethyl-2-oxa-7-azabicyclo[3.3.0]octan,
[α]⁶ : + 11,9 (c = 0,7, Methanol).

Durch Behandlung von (1R,5R)-1-tert.-Butoxycarbonylaminomethyl-2-oxa-7-azabicyclo[3.3.0]octan mit konzentrierter Salzsäure bei Raumtemperatur erhält man (1S,5R)-1-Aminomethyl-2-oxa-7-azabicyclo[3.3.0]octan-Hydrochlorid.

### Beispiel Z 13

### 8-Cyano-1-cyclopropyl-6,7-difluor-1,4-dihydro-4-oxo-3-chinolin-carbonsäureethylester

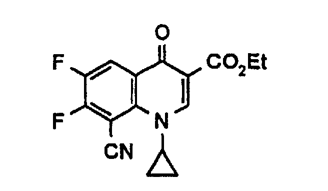

A. 3-Brom-2,4,5-trifluor-benzoesäuremethylester: Zu einer Mischung aus 1460 ml Methanol und 340 g Triethylamin werden unter Eiskühlung 772 g 3-Brom-2,4,5-trifluor-benzoylfluorid getropft. Es wird 1 Stunde bei Raumtemperatur nachgerührt.
   Das Reaktionsgemsich wird eingeengt, der Rückstand in Wasser und Methylenchlorid aufgenommen und die wäßrige Phase nochmals mit Methylenchlorid ausgeschüttelt.
   Nach Trocknen der organischen Phase über Natriumsulfat wird eingeengt und der Rückstand im Vakuum destilliert. Man erhält 752,4 g 3-Brom-2,4,5-trifluor-benzoesäuremethylester vom Siedepunkt 122°C/20 mbar.
B. 3-Cyano-2,4,5-trifluor-benzoesäuremethylester: 269 g 3-Brom-2,4,5-trifluorbenzoesäuremethylester und 108 g Kupfercyanid werden in 400 ml Dimethylformamid während 5 Stunden zum Rückfluß erhitzt. Anschließend werden im Vakuum alle flüchtigen Bestandteile des Reaktionsgemisches abdestilliert. Das Destillat wird dann an einer Kolonne fraktioniert. Man erhält 133 g 3-Cyano-2,4,5-trifluor-benzoesäuremethylester vom Siedepunkt 88-89°C/0,01 mbar.
C. 3-Cyano-2,4,5-trifluor-benzoesäure: Eine Lösung von 156 g 3-Cyano-2,4,5-trifluor-benzoesäuremethylester in 960 ml Eisessig, 140 ml Wasser und 69 ml konzentrierter Schwefelsäure wird 8 Stunden zum Rückfluß erhitzt. Anschließend wird die Essigsäure im Vakuum weitgehend abdestilliert und der Rückstand mit Wasser versetzt. Der ausgefallene Feststoff wird abgesaugt, mit Wasser gewaschen und getrocknet. Man erhält 118,6 g 3-Cyano-2,4,5-trifluor-benzoesäure als weißen Feststoff vom Schmelzpunkt 187-190°C.
D. 3-Cyano-2,4,5-trifluor-benzoesäurechlorid: 111 g 3-Cyano-2,4,5-trifluor-benzoesäure und 84 g Oxalylchlorid werden in 930 ml trockenem Methylenchlorid unter Zusatz einiger Tropfen Dimethylformamid 5 Stunden bei Raumtemperatur gerührt. Anschließend wird das Methylenchlorid abgezogen und der Rückstand im Vakuum destilliert. Man erhält 117,6 g 3-Cyano-2,4,5-trifluor-benzoylchlorid als gelbes Öl.
E. 2-(3-Cyano-2,4,5-trifluor-benzoyl)-3-dimethylamino-acrylsäureethylester: Zu einer Lösung von 36,5 g 3-Dimethylamino-acrylsäureethylester und 26,5 g Triethylamin in 140 ml Toluol wird eine Lösung von 55 g 3-Cyano-2,4,5-trifluor-benzoesäurechlorid in 50 ml Toluol so zugetropft, daß die Temperatur zwischen 50 und 55°C bleibt. Dann wird noch 2 Stunden bei 50°C nachgerührt. Das Reaktionsgemisch wird im Vakuum eingeengt und ohne weitere Aufarbeitung in die nächste Stufe eingesetzt.
F. 2-(3-Cyano-2,4,5-trifluor-benzoyl)-3-cyclopropylamino-acrylsäureethylester: Zu dem Reaktionsprodukt aus der Stufe E werden bei 20°C 30 g Eisessig getropft. Anschließend wird eine Lösung von 15,75 g Cyclopropylamin in 30 ml Toluol zugetropft. Das Gemisch wird 1 Stunde bei 30°C gerührt. Dann gibt man 200 ml Wasser hinzu, verrührt 15 Minuten, trennt die organische Phase ab und schüttelt sie nochmals mit 100 ml Wasser aus. Dann wird die organische Phase über Natriumsulfat getrocknet und im Vakuum eingeengt. Das so erhaltene Rohprodukt wird ohne weitere Aufarbeitung in die nächste Stufe eingesetzt.
G. 8-Cyano-1-cyclopropyl-6,7-difluor-1,4-dihydro-4-oxo-3-chinolincarbonsäureethylester: Das Reaktionsprodukt aus der Stufe f und 27,6 g Kaliumcarbonat werden in 80 ml Dimethylformamid 16 Stunden bei Raumtemperatur gerührt. Das Reaktionsgemisch wird dann in 750 ml Eiswasser gegeben, der Feststoff abgesaugt und mit 80 ml kaltem Methanol gewaschen. Nach Trocknen erhält man 47 g 8-Cyano-1-cyclopropyl-6,7-difluor-1,4-dihydro-4-oxo-3-chinolincarbonsäureethylester vom Schmelzpunkt 209-211°C.

### Beispiel Z 14

### 2,4-Dichlor-5-fluor-1,3-dimethylbenzol

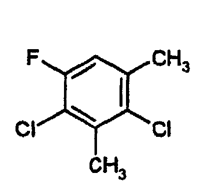

### a) lösungsmittelfrei

In 124 g 3,5-Dimethyl-fluorbenzol werden 1 g wasserfreies Eisen(III)-chlorid vorgelegt und mit der Geschwindigkeit Chlor eingeleitet (ca. 4 h), mit der die Reaktion abläuft. Diese ist anfangs etwas exotherm (Temperaturanstieg von 24 auf 32°C) und wird durch Kühlung unter 30°C gehalten. Nach Zufuhr von 120 g Chlor wird der Ansatz fest. Nach GC-Analyse sind 33,4 % Monochlorverbindung, 58,4 % gewünschtes Produkt und 5 % überchlorierte Verbindungen entstanden. Der Chlorwasserstoff wird abgezogen und die Reaktiosmischung anschließend im Wasserstrahlvakuum an einer Kolonne destilliert:

Im Vorlauf werden bei 72-74°C/22 mbar 49 g 2-Chlor-5-fluor-1,3-dimethylbenzol erhalten. Nach einem Zwischenlauf von 5 g gehen bei 105°C/22 mbar 75 g 2,4-Dichlor-5-fluor-1,3-dimethylbenzol über; Schmelzbereich: 64 - 65°C.

### b) in 1,2-Dichlorethan

1 kg 3,5-Dimethyl-fluorbenzol und 15 g wasserfreies Eisen(III)-chlorid werden in 11 1,2-Dichlorethan vorgelegt und in dem Maße Chlor eingeleitet, wie die Reaktion fortschreitet (ca. 4 h). Die Reaktion ist anfangs exotherm (Temperaturanstieg von 24 auf 32°C) und wird durch Kühlung unter 30°C gehalten. Nach dem Einleiten von 1200 g Chlor sind laut GC-Analyse 4 % Monochlorverbindung, 81,1 % gewünschtes Produkt und 13,3 % überchlorierte Verbindungen entstanden. Nach Abdestillation des Lösungsmittels und des Chlorwasserstoffs wird im Wasserstrahlvakuum an einer Kolonne destilliert:

Im Vorlauf werden 40 g 2-Chlor-5-fluor-1,3-dimethylbenzol erhalten. Nach wenig Zwischenlauf gehen bei 127 - 128°C/50 mbar 1115g 2,4-Dichlor-5-fluor-1,3-dimethylbenzol über.

### Beispiel Z 15

### 2,4-Dichlor-5-fluor-3-dichlormethyl-1-trichlormethylbenzol

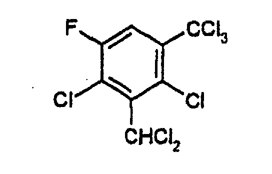

In einer Photochlorierungsapparatur mit Chloreinleitung und Ableitung für den Chlorwasserstoff zu einem Wäscher sowie einer Lichtquelle in der Nähe des Chloreinleitungsrohrs werden 1890 g 2,4-Dichlor-5-fluor-1,3-dimethylbenzol vorgelegt und bei 140 bis 150°C Chlor eindosiert. Innerhalb 30 h werden 3850 g Chlor eingeleitet. Der Gehalt an gewünschtem Produkt beträgt nach GC-Analyse 71,1 %; der Anteil an minderchlorierten Verbindungen 27,7 %.

Die Destillaton über eine 60 cm-Kolonne mit Wilson-Spiralen liefert einen Vorlauf von 1142 g, der erneut in die Chlorierung eingesetzt werden kann. Der Hauptlauf bei 160-168°C/0,2 mbar liefert 2200 g 2,4-Dichlor-5-fluor-3-dichlormethyl-1-trichlormethylbenzol mit einem Schmelzbereich von 74-76°C. Nach Umkristallisieren einer Probe aus Methanol beträgt der Schmelzpunkt 81-82°C.

### Beispiel Z 16

### 2,4-Dichlor-5-fluor-3-formyl-benzoesäure

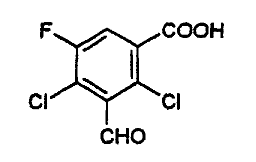

In einer Rührapparatur mit Gasableitung werden 2500 ml 95%-ige Schwefelsäure bei 70°C vorgelegt und unter Rühren 500 g aufgeschmolzenes 2,4-Dichlor-5-fluor-3-dichlormethyl-1-trichlormethylbenzol zugetropft. Es setzt nach kurzer Zeit Chlorwasserstoffentwicklung ein. Man dosiert während 2 h ein und rührt bis zum Ende der Gasentwicklung nach. Nach Abkühlen auf 20°C wird der Ansatz auf 4 kg Eis ausgetragen und der ausgefallene Feststoff abgesaugt. Das Produkt wird mit Wasser nachgewaschen und getrocknet.
Ausbeute: 310 g,
Schmelzbereich: 172-174°C

### Beispiel Z 17

### 2,4-Dichlor-5-fluor-3-N-hydroxyiminomethyl-benzoesäure

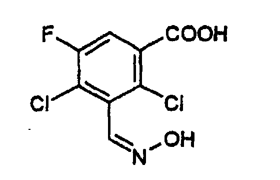

In einer Rührapparatur werden 80 g Hydroxylammoniumchlorid in 500 ml Ethanol vorgelegt und 200 ml 45 %-ige Natronlauge zugetropft und anschließend bei 40-45°C 200 g 2,4-Dichlor-5-fluor-3-formyl-benzoesäure eingetragen. Die Reaktion ist leicht exotherm und es wird 5 h bei 60°C nachgerührt. Nach Abkühlen auf Raumtemperatur wird durch Zutropfen von Salzsäure auf pH < 3 gestellt, das Produkt in tert.-Butylmethyl-ether aufgenommen, die organische Phase abgetrennt und das Lösungsmittels abdestilliert. Als Rückstand erhält man 185 g 2,4-Dichlor-5-fluor-3-N-hydroxyiminomethyl-benzoesäure; Schmelzbereich: 190-194°C.

### Beispiel Z 18

### 2,4-Dichlor-3-cyano-5-fluor-benzoylchlorid

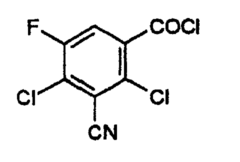

In einer Rührapparatur mit Dosiervorrichtung und Gasableitung über einen Rückflußkühler zu einem Wäscher werden 600 ml Thionylchlorid vorgelegt und bei 20°C 210 g 2,4-Dichlor-5-fluor-3-N-hydroxyiminomethyl-benzoesäure in dem Maße eingetragen, wie sich Chlorwasserstoff und Schwefeldioxid entwickelt. Nach der Zugabe wird die Mischung bis zum Ende der Gasentwicklung unter Rückfluß erhitzt. Anschließend wird destilliert und im Siedebereich von 142-145°C/10 mbar 149 g 2,4-Dichlor-3-cyano-5-fluor-benzoylchlorid erhalten (Gehalt nach GC; 98,1 %); Schmelzbereich: 73-75°C.

### Beispiel Z 19

### 3 -Cyano-2,4,5-trifluor-benzoylfluorid

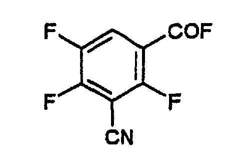

50g Kaliumfluorid werden in 120 ml Tetramethylensulfon suspendiert und bei 15 mbar zur Trocknung andestilliert (ca. 20 ml). Anschließend werden 50,4 g 2,4-Dichlor-3-cyano-5-fluor-benzoylchlorid zugefügt und unter Feuchtigkeitsausschluß für 12 Stunden bei 180°C Innentemperatur gerührt. Durch Vakuumdestillation werden 32,9 g 3-Cyano-2,4,5-trifluor-benzoylfluorid im Siedebereich von 98-100°C/12 mbar erhalten.

### Beispiel Z 20

### 3-Cyano-2,4,5-trifluor-benzoylchlorid

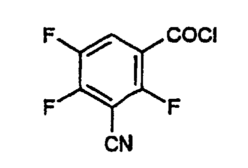

76,6 g 3-Cyano-2,4,5-trifluor-benzoylfluorid werden zusammen mit 1 g wasserfreiem Aluminiumchlorid bei 60-65°C vorgelegt und dann im Zuge der Gasentwicklung 25 g Siliciumtetrachlorid zugetropft. Nach Ende der Gasentwicklung bei 65°C wird im Vakuum destilliert. Im Siedebereich von 120-122°C/14 mbar gehen 73,2 g 3-Cyano-2,4,5-trifluor-benzoylchlorid über.

### Beispiel Z 21

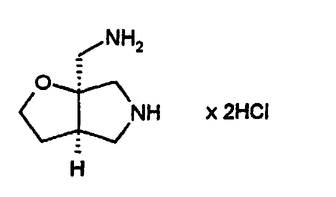

0,5 g (2,07 mmol) (1R,5R)-1-tert.-Butoxycarbonylaminomethyl-2-oxa-7-azabicyclo[3.3.0]octan werden in 30 ml Ethanol mit 13 ml etwa 3,5 n Salzsäure versetzt und 4 Stunden bei Raumtemperatur gerührt. Die Mischung wird eingeengt und das Salz isoliert.

Ausbeute: 0,3 g (67,5 % der Theorie) (1S,5R)-1-Aminomethyl-2-oxa-7-azabicyclo[3.3.0]octan-Dihydrochlorid,
Schmelzpunkt: 284°C,
[α]⁴: + 16,5° (c = 1, Methanol).

Entsprechend erhält man auch (1R,5S)-1-Aminomethyl-2-oxa-7-azabicyclo[3.3.0]octan-Dihydrochlorid sowie (1SR,5RS)-1-Aminomethyl-2-oxa-7-azabicyclo[3.3.0]-octan-Dihydrochlorid.

### Herstellung der Wirkstoffe

### Beispiel 1

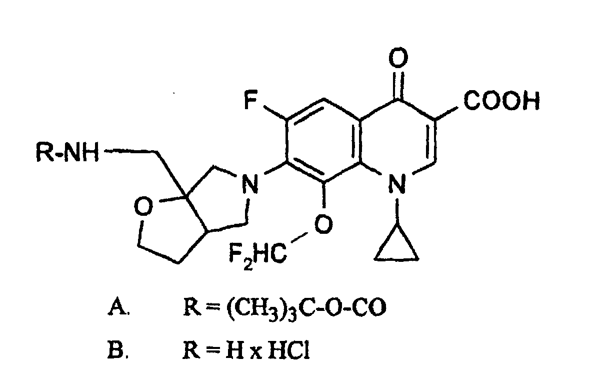

A: Eine Mischung von 332 mg (1 mmol) 1-Cyclopropyl-8-difluormethoxy-6,7-difluor-1,4-dihydro-4-oxo-3-chinolincarbonsäure in 4 ml Acetonitril und 2 ml Dimethylformamid mit 112 mg (1 mmol) 1,4-Diazabicyclo[2.2.2]octan und 267 mg (1,1 mmol) 1-tert.-Butoxycarbonylaminomethyl-2-oxa-7-aza-bicyclo[3.3.0]octan wird 1 Stunde unter Rückfluß erhitzt. Die Suspension wird bei 70 °C/15 mbar eingeengt, der Rückstand mit etwas Wasser versetzt und 30 Minuten im Ultraschallbad behandelt. Der ungelöste Niederschlag wird abgesaugt, mit Wasser gewaschen und bei 90°C im Hochvakuum getrocknet.
   Ausbeute: 487 mg (88 % der Theorie) 7-(1-tert.-Butoxycarbonylaminomethyl-2-oxa-7-aza-bicyclo[3.3.0]oct-7-yl)-1-cyclopropyl-8-difluormethoxy-6-fluor-1,4-dihydro-4-oxo-3-chinolincarbonsäure,
   Schmelzpunkt: 215-217 C (unter Zersetzung).
B: 476 mg (0,86 mmol) 7-(l-tert.-Butoxycarbonylaminomethyl-2-oxa-7-aza-bicyclo[3.3.0]oct-7-yl)-1-cyclopropyl-8-difluormethoxy-6-fluor-1,4-dihydro-4-oxo-3-chinolincarbonsäure werden in 5 ml halbkonzentrierter Salzsäure in der Wärme gelöst, die Lösung filtriert und bei 60 C/15 mbar eingeengt. Der Rückstand wird mit etwas Ethanol versetzt, erneut eingeengt, das Salz isoliert und bei 90 C im Hochvakuum getrocknet.
Ausbeute: 385 mg (91 % der Theorie) 7-(1-Aminomethyl-2-oxa-7-aza-bicyclo[3.3.0]-oct-7-yl)-1-cyclopropyl-8-difluormethoxy-6-fluor-1,4-dihydro-4-oxo-3-chinolincarbonsäure-Hydrochlorid,
   Schmelzpunkt: 182-185 C (unter Zersetzung).
AA. Analog Stufe A wird mit (+)-(1R,5R)-1-tert.-Butoxycarbonylaminomethyl-2-oxa-7-aza-bicyclo[3.3.0]octan zu (+)-(1R,5R)-7-(1-tert.-Butoxycarbonylaminomethyl-2-oxa-7-aza-bicyclo[3.3.0]oct-7-yl)-1-cyclopropyl-8-difluormethoxy-6-fluor-1,4-dihydro-4-oxo-3-chinolincarbonsäure umgesetzt.
   Schmelzpunkt: 166-167°C (unter Zersetzung),
   [α]⁵: +26° (c = 0,25, DMF).
BA. Analog Stufe B wird das Produkt aus Stufe AA zu (-)-(1S,5R)-7-(1-Aminomethyl-2-oxa-7-aza-bicyclo[3.3.0]oct-7-yl)-1-cyclopropyl-8-difluormethoxy-6-fluor-1,4-dihydro-4-oxo-3-chinolincarbonsäure-Hydrochlorid umgesetzt.
   Schmelzpunkt: 194-197°C (unter Zersetzung),
   [α]⁵: -21° (c = 0,5, DMF).
AB. Analog Stufe A wird mit (-)-(1S,5S)-1-tert.-Butoxycarbonylaminomethyl-2-oxa-7-aza-bicyclo[3.3.0]octan zu (-)-(1S,5S)-7-(1-tert.-Butoxycarbonylaminomethyl-2-oxa-7-aza-bicyclo[3.3.0]oct-7-yl)-1-cyclopropyl-8-difluormethoxy-6-fluor-1,4-dihydro-4-oxo-3-chinolincarbonsäure umgesetzt.
   Schmelzpunkt: 166-167°C (unter Zersetzung),
   [α]⁵: -27° (c = 0,125, DMF).
BB. Analog Stufe B wird das Produkt aus Stufe AB zu (+)-(1R,5S)-7-(1-Aminomethyl-2-oxa-7-aza-bicyclo[3.3.0]oct-7-yl)-1-cyclopropyl-8-difluormethoxy-6-fluor-1,4-dihydro-4-oxo-3-chinolincarbonsäure-Hydrochlorid umgesetzt.
   Schmelzpunkt: 194-197°C (unter Zersetzung),
   [α]⁵: + 19° (c = 0,5, DMF)

### Beispiel 2

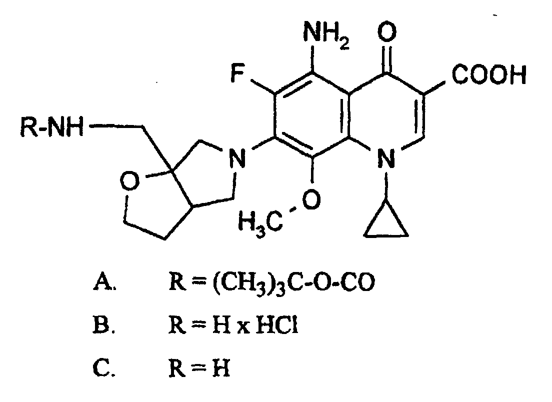

A: Analog Beispiel 1A setzt man mit 5-Amino-1-cyclopropyl-6,7-difluor-1,4-dihydro-8-methoxy-4-oxo-3-chinolincarbonsäure zu 5-Amino-7-(1-tert.-butoxycarbonylaminomethyl-2-oxa-7-aza-bicyclo[3.3.0]oct-7-yl)-1-cyclopropyl-6-fluor-1,4-dihydro-8-methoxy-4-oxo-3-chinolincarbonsäure (77 % der Theorie) vom Schmelzpunkt 219-221°C (unter Zersetzung) (nach chromatographischer Reinigung an Kieselgel mit Dichlormethan/Methanol/17 % wäßriges Ammoniak 150/20/1 als Laufmittel) um.
B: Analog Beispiel 1 B werden 405 mg (0,76 mmol) des Produkts aus Stufe A mit 7 ml halbkonzentrierter Salzsäure umgesetzt.
   Ausbeute: 244 mg (69 % der Theorie) 5-Amino-7-(1-aminomethyl-2-oxa-7-aza-bicyclo[3.3.0]oct-7-yl)-1-cyclopropyl-6-fluor-1,4-dihydro-8-methoxy-4-oxo-3-chinolincarbonsäure-Hydrochlorid,
   Schmelzpunkt: 242-244 C (unter Zersetzung).
   FAB-MS: m/e 433 [(M+H)⁺].
C: 235 mg (0,44 mmol) des Produkts aus Stufe A werden in 20 ml Dichlormethan bei Raumtemperatur gelöst und mit 1,4 ml Trifluoressigsäure versetzt. Man rührt 3 Stunden bei Raumtemperatur, engt ein und dampft den Rückstand zweimal mit Ethanol ab. Man erhält 148 mg 5-Amino-7-(1-aminomethyl-2-oxa-7-aza-bicyclo[3.3.0]oct7-yl)-1-cyclopropyl-6-fluor-1,4-dihydro-8-methoxy-4-oxo-3-chinolincarbonsäure-Trifluoracetat als Rohprodukt, das an 40 g Kieselgel mit Dichlormethan/Methanol/17 % wäßriges Ammoniak (150/20/1) als Laufmittel gereinigt wird, wobei das Betain anfällt.

Ausbeute: 69 mg (36 % der Theorie) 5-Amino-7-(1-amiomethyl-2-oxa-7-aza-bicyclo[3.3.0]oct-7-yl)-1-cyclopropyl-6-fluor-1,4-dihydro-8-methoxy-4-oxo-3-chinolincarbonsaure,
Schmelzpunkt: 209-211 °C (unter Zersetzung).

### Beispiel 3

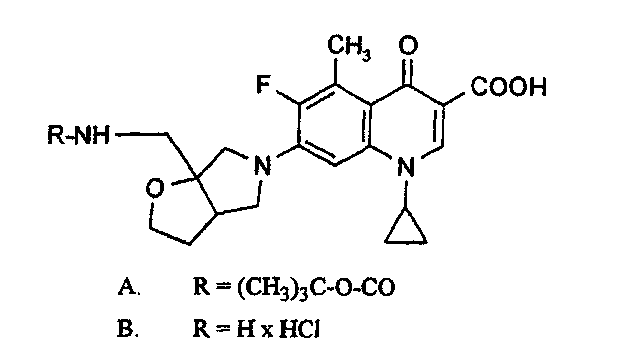

A: Analog Beispiel 1A setzt man mit 1-Cyclopropyl-6,7-difluor-1,4-dihydro-5-methyl-4-oxo-3-chinolincarbonsäure zu 7-(1-tert.-Butoxycarbonylaminomethyl-2-oxa-7-aza-bicyclo[3.3.0]oct7-yl)-1-cyclopropyl-6-fluor-1,4-dihydro-5-methyl-4-oxo-3-chinolincarbonsäure (76 % der Theorie) um.
B: Analog Beispiel 1B wird das Produkt aus der Stufe A mit halbkonzentrierter Salzsäure zu 7-(1-Aminomethyl-2-oxa-7-aza-bicyclo[3.3.0]oct-7-yl)-1-cyclopropyl-6-fluor-1,4-dihydro-5-methyl-4-oxo-3-chinolincarbonsäure-Hydrochlorid vom Schmelzpunkt 251-253 C (unter Zersetzung) umgesetzt.

### Beispiel 4

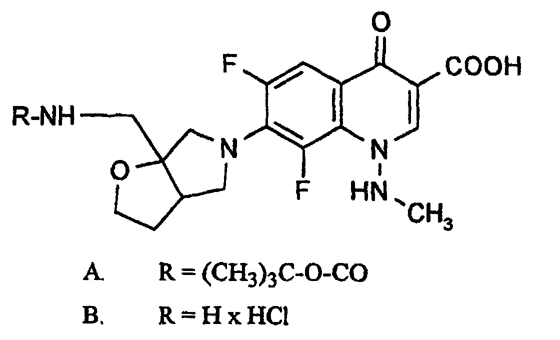

A: Analog Beispiel 1A setzt man mit 6,7,8-Trifluor-1,4-dihydro-1-methylamino-4-oxo-3-chinolincarbonsäure zu 7-(1-tert.-Butoxycarbonylaminomethyl-2-oxa-7-azabicyclo[3.3.0]oct-7-yl)-6,8-difluor-1,4-dihydro-1-methylamino-4-oxo-3-chinolincarbonsäure (68 % der Theorie) vom Schmelzpunkt 225-227 C (unter Zersetzung) um.
B: Analog Beispiel 1B wird das Produkt aus der Stufe A mit halbkonzentrierter Salzsäure in 65 %-iger Ausbeute zu 7-(1-Aminomethyl-2-oxa-7-aza-bicyclo[3.3.0]oct-7-yl)-6,8-difluor-1,4-dihydro-1-methylamino-4-oxo-3-chinolincarbonsäure-Hydrochlorid vom Schmelzpunkt 177-179 C (unter Zersetzung) umgesetzt.
   FAB-MS: m/e: 395 [(M+H)⁺], 366.

### Beispiel 5

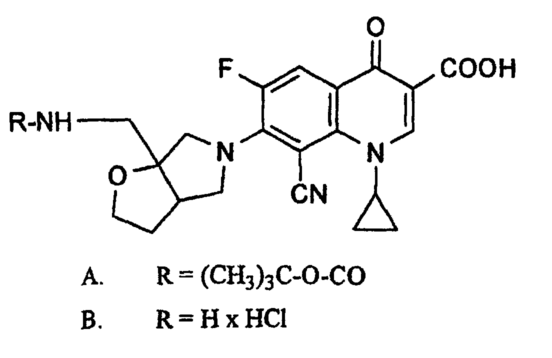

A: Analog Beispiel 1A setzt man mit 7-Chlor-8-cyano-1-cyclopropyl-6-fluor-1,4-dihydro-4-oxo-3-chinolincarbonsäure (europäisches Patent 276 700) zu 7-(1-tert.-Butoxycarbonylaminomethyl-2-oxa-7-aza-bicyclo[3.3.0]oct-7-yl)-8-cyano-1-cyclopropyl-6-fluor-1,4-dihydro-4-oxo-3-chinolincarbonsäure um.
B: Analog Beispiel 1B wird das Produkt aus der Stufe A mit halbkonzentrierter Salzsäure zu 7-(1-Aminomethyl-2-oxa-7-aza-bicyclo[3.3.0]oct-7-yl)-8-cyano-1-cyclopropyl-6-fluor-1,4-dihydro-4-oxo-3-chinolincarbonsäure-Hydrochlorid vom Schmelzpunkt 241 C (unter Zersetzung) umgesetzt.
C: Analog der Umsetzung in Stufe A und B erhält man mit (+)-1-tert.-Butoxycarbonylaminomethyl-2-oxa-7-aza-bicyclo[3.3.0]octan (+)-7-(1-Aminomethyl-2-oxa-7-azabicyclo[3.3.0]oct-7-yl)-8-cyano-1-cyclopropyl-6-fluor-1,4-dihydro-4-oxo-3-chinolincarbonsäure-Hydrochlorid,
   Schmelzpunkt: 293°C (unter Zersetzung),
   [α]⁴ : +50,6° (c = 0,7, Methanol).
D: Analog der Umsetzung in Stufe A und B erhält man mit (-)-1-tert.-Butoxycarbonylaminometbyl-2-oxa-7-aza-bicyclo[3.3.0]octan (-)-7-(1-Aminomethyl-2-oxa-7-azabicyclo[3.3.0]oct-7-yl)-8-cyano-1-cyclopropyl-6-fluor-1,4-dihydro-4-oxo-3-chinolincarbonsäure-Hydrochlorid,
   Schmelzpunkt: 292°C (unter Zersetzung),
   [α]⁴ : -49° (c = 1, Methanol).
E: Eine Lösung von 5,4 g (0,224 mol) (1R,5R)-1-tert.-Butoxycarbonylaminomethyl-2-oxa-7-azabicyclo[3.3.0]octan, 6,5 g (0,204 mol) 8-Cyano-1-cyclopropyl-6,7-difluor-1,4-dihydro-4-oxo-chinolincarbonsäureethylester und 4,6 g (0,459 mol) Triethylamin in 300 ml absolutem Acetonitril wird über Nacht bei Raumtemperatur gerührt. Anschließend wird im Vakuum eingeengt und der Rückstand über Kieselgel chromatographiert (Dichlormethan/Methanol, 99:1 95:5). Man erhält 9,2 g (83,6% der Theorie) (+)-7-[(1R,5R)-1-tert.-Butoxycarbonylaminomethyl-2-oxa-7-aza-bicyclo[3.3.0]-oct-7-yl]-8-cyano- 1-cyclopropyl-6-fluor-1,4-dihydro-4-oxo-chinolincarbonsäureethylester,
   [α]⁴ : + 81,1° (c = 1,5, Dichlormethan).

9,1 g (0,169 mol) (+)-7-[(1R,5R)-1-tert.-Butoxycarbonylaminomethyl-2-oxa-7-aza-bicyclo[3.3.0]-oct-7-yl]-8-cyano-1-cyclopropyl-6-fluor-1,4-dihydro-4-oxochinolincarbonsäureethylester werden mit 150 ml 10%iger Salzsäure versetzt. Das Gemisch wird 1 Stunde unter Rückfluß erhitzt und anschließend auf Raumtemperatur abgekühlt. Die farblosen Kristalle werden abfiltriert und mit wenig absolutem Isopropanol gewaschen.

Ausbeute: 6,9 g (91% der Theorie) (+)-7-[(1S,5R)-1-Aminomethyl-2-oxa-7-azabicyclo[3.3.0]-oct-7-yl]-8-cyano-1-cyclopropyl-6-fluor-1,4-dihydro-4-oxo-chinolincarbonsäure-Hydrochlorid,
Schmelzpunkt.: 293 C
[α]⁴ : + 50,6 (c = 0,8 in Methanol).

### Beispiel 6

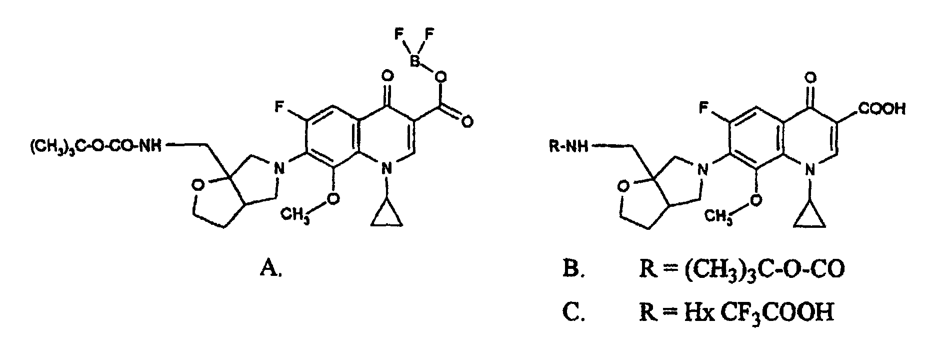

A: 172 mg (0,5 mmol) 1-Cyclopropyl-6,7-difluor-1,4-dihydro-8-methoxy-4-oxo-3-chinolincarbonsäure-BF₂-Chelat werden in 5 ml Acetonitril/Dimethylformamid (1:1) mit 150 mg (0,62 mmol) 1-tert.-Butoxycarbonylaminomethyl-2-oxa-7-aza-bicyclo[3.3.0]octan und 40 mg (0,38 mmol) 1,4-Diazabicyclo[2.2.2]octan versetzt und 12 Stunden auf 40 °C erhitzt. Der Ansatz wird eingeengt und kann als Rohprodukt in Stufe B eingesetzt werden. Er kann aber auch zur Charakterisierung der Zwischenstufe chromatographisch an Kieselgel (Laufmittel: Dichlormethan/Methanol 95:5) gereinigt werden. Hierbei werden 128 mg 7-(1-tert.-Butoxycarbonylaminomethyl-2-oxa-7-aza-bicyclo[3.3.0]oct-7-yl)-1-cyclopropyl-6-fluor-1,4-dihydro-8-methoxy-4-oxo-3-chinolincarbonsaure-BF₂-Chelat als gelber Feststoff isoliert; FAB-MS: m/e 566 [(M+H)⁺], 546 [(M-F⁻)⁺].
B: Der aus Stufe A erhaltene Eindampfrückstand wird in 20 ml Dichlormethan/Methanol (1:1) aufgenommen und nach Zugabe von 2,6 ml Triethylamin 10 Stunden unter Rückfluß erhitzt. Die Mischung wird eingeengt, der Rückstand in 4 ml Wasser aufgenommen und mit 3 ml 2n-HCl versetzt. Der ausgefallene Niederschlag wird abgesaugt, mit 20 ml Wasser gewaschen und bei 80°C im Hochvakuum getrocknet. Man erhält 210 mg 7-(1-tert.-Butoxycarbonylaminomethyl-2-oxa-7-aza-bicyclo[3.3.0]oct-7-yl)-1-cyclopropyl-6-fluor-1,4-dihydro-8-methoxy-4-oxo-3-chinolincarbonsäure; ¹H-NMR (400 MHz; DMSO): δ 3,63 ppm s (OCH₃).
C: 206 mg (0,4 mmol) des Produkts aus Stufe B werden in 13 ml Dichlormethan gelöst und mit 3,4 ml Trifluoressigsäure versetzt. Man rührt 30 Minuten bei 25°C und engt die Lösung im Vakuum ein. Der Rückstand wird mehrmals mit absolutem Ethanol verrührt, der Niederschlag abgesaugt, gut mit Ethanol gewaschen und bei 80°C im Hochvakuum getrocknet.
   Ausbeute: 114 mg mg (54 % der Theorie) 7-(1-Aminomethyl-2-oxa-7-aza-bicyclo[3.3.0]oct-7-yl)-1-cyclopropyl-6-fluor-1,4-dihydro-8-methoxy-4-oxo-3-chinolincarbonsäure-Trifluoracetat,
   Schmelzpunkt: 215-216°C (unter Zersetzung).

### Beispiel 7

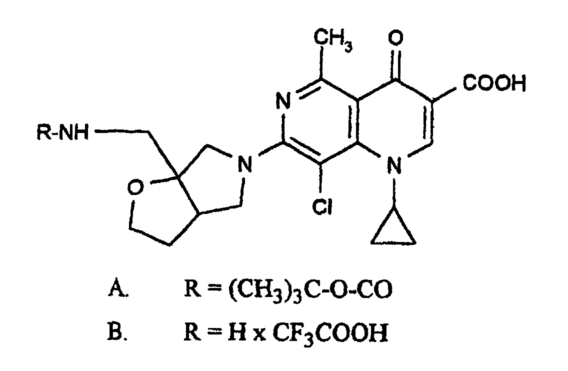

A: 313 mg (1 mmol) 7,8-Dichlor-1-cyclopropyl-1,4-dihydro-5-methyl-4-oxo-1,6-naphthydin-3-carbonsäure werden in 5 ml Acetonitril mit 168 mg (1,5 mmol) 1,4-Diazabicyclo[2.2.2]octan und 266 mg (1,1 mmol) 1-tert.-Butoxycarbonylaminomethyl-2-oxa-7-aza-bicyclo[3.3.0]octan versetzt und 2 Stunden bei 50 C gerührt. Die Mischung wird eingeengt, der Rückstand mit Wasser behandelt, der Niederschlag abgesaugt, mit Wasser gewaschen und bei 90°C im Hochvakuum getrocknet.
   Ausbeute: 272 mg (52,5 % der Theorie) 7-(1-tert.-Butoxycarbonylaminomethyl-2-oxa-7-aza-bicyclo[3.3.0]oct-7-yl)-8-chlor-1-cyclopropyl-1,4-dihydro-5-methyl-4-oxo-1,6-naphthyridin-3-carbonsäure,
   Schmelzpunkt: 209-210°C (unter Zersetzung).
B: 150 mg (0,29 mmol) des Produkts aus Stufe A werden in 2 ml Dichlormethan mit 1,5 ml Trifluoressigsäure versetzt und 45 Minuten bei 25°C gerührt. Die Lösung wird eingeengt und der Rückstand wiederholt mit Dichlormethan versetzt und erneut eingeengt. Das erhaltene Salz wird isoliert und bei 70°C im Hochvakuum getrocknet.
   Ausbeute: 68 mg (45 % der Theorie) 7-(1-Aminomethyl-2-oxa-7-aza-bicyclo[3.3.0]-oct-7-y1)-8-Chlor-1-cyclopropyl-1,4-dihydro-5-methyl-4-oxo-1,6-naphthyridin-3-carbonsäure-Trifluoracetat,
   Schmelzpunkt: 125-128°C (unter Zersetzung).
   Massenspektrum (ESI): m/e 419 [(M+H)⁺].

### Beispiel 8

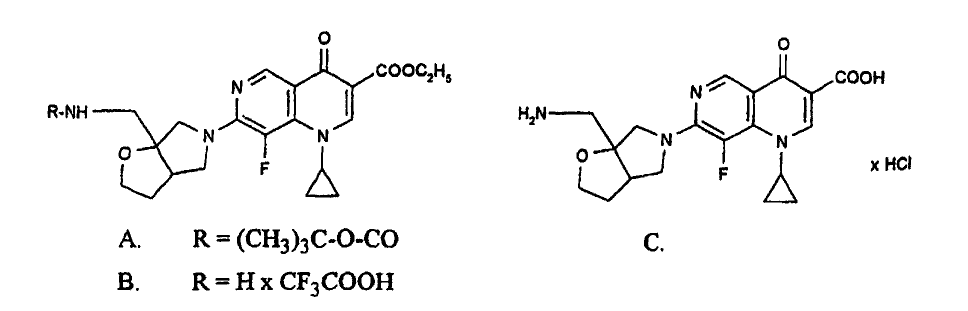

A: Analog Beispiel 7A setzt man mit 7-Chlor-1-cyclopropyl-8-fluor-1,4-dihydro-4-oxo-1,6-naphthyridin-3-carbonsäureethylester in 20 %-iger Ausbeute zu 7-(1-tert.-Butoxycarbonylaminomethyl-2-oxa-7-aza-bicyclo[3.3.0]oct-7-yl)-1-cyclopropyl-8-fluor-1,4-dihydro-4-oxo-1,6-naphthyridin-3-carbonsäureethylester vom Schmelzpunkt 146-148 C (unter Zersetzung) um.
B: Analog Beispiel 7B wird das Produkt aus der Stufe A mit Trifluoressigsäure zu 7-(1-Aminomethyl-2-oxa-7-aza-bicyclo[3.3.0]oct-7-yl)-1-cyclopropyl-8-fluor-1,4-dihydro-4-oxo-1,6-naphthyridin-3-carbonsäureethylester-Trifluoracetat vom Schmelzpunkt 199-200 C (unter Zersetzung) umgesetzt.
C: 136 mg (0,25 mmol) des Produkts aus Stufe B werden mit 10 ml halbkonzentrierter Salzsäure versetzt und ca. 30 Minuten unter Rückfluß erhitzt. Man engt ein und behandelt den Rückstand mit Ethanol. Der Feststoff wird abgesaugt und bei 80°C im Hochvakuum getrocknet.
   Ausbeute: 97 mg (91 % der Theorie) 7-(1-Aminomethyl-2-oxa-7-aza-bicyclo[3.3.0]-oct-7-yl)-1-cyclopropyl-8-fluor-1,4-dihydro-4-oxo-1,6-naphthyridin-3-carbonsäure-Hydrochlorid,
   Schmelzpunkt: 301-304°C (unter Zersetzung).

### Beispiel 9

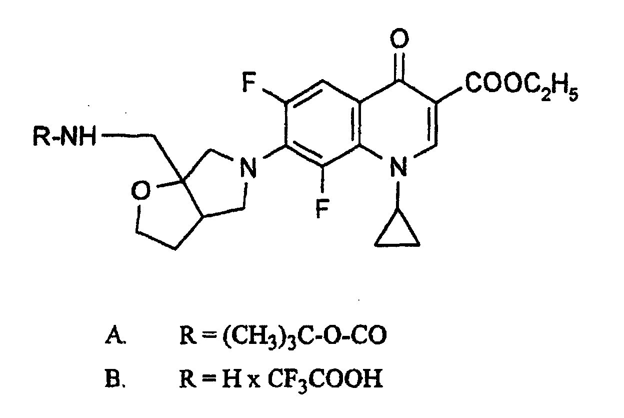

A: Analog Beispiel 1 A wird mit 1-Cyclopropyl-6,7,8-trifluor-1,4-dihydro-4-oxo-3-chinolincarbonsäureethylester zu 7-(1-tert.-Butoxycarbonylaminomethyl-2-oxa-7-azabicyclo[3.3.0]oct-7-yl)-1-cyclopropyl-6,8-difluor-1,4-dihydro-4-oxo-3-chinolincarbonsäureethylester umgesetzt.
B. 1,2 g des Produkts aus Stufe A werden in 10 ml Trifluoressigsäure bei 25°C 5 Minuten gerührt, danach eingeengt und der Rückstand mit Ether verrührt. Der erhaltene Feststoff wird in Dichlormethan/Methanol (95:5) an wenig Kieselgel chromatographisch gereinigt.
   Ausbeute: 215 mg (17 % der Theorie) 7-(1-Aminomethyl-2-oxa-7-aza-bicyclo[3.3.0]oct-7-yl)-1-cyclopropyl-6,8-difluor-1,4-dihydro-4-oxo-3-chinolincarbonsäureethylester-Trifluoracetat,
   Schmelzpunkt: 139-141°C.

### Beispiel 10

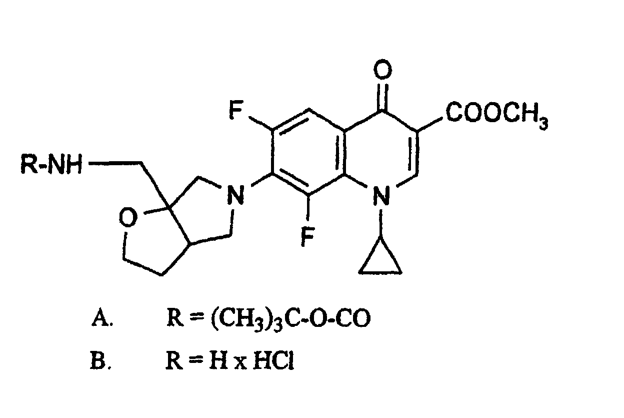

A. 4,96 g (10 mmol) 7-(1-tert.-Butoxycarbonylaminomethyl-2-oxa-7-aza-bicyclo[3.3.0]oct-7-yl)-1-cyclopropyl-6,8-difluor-1,4-dihydro-4-oxo-3-chinolincarbonsäure werden in 200 ml Tetrahydrofuran/Wasser (1:1) bei 25°C suspendiert, mit 2,3 g (7 mmol) Cäsiumcarbonat versetzt und ca. 10 Minuten im Ultraschallbad behandelt, wobei eine klare Lösung erhalten wird. Bei 30°C/15 mbar wird weitgehend eingeengt und die Restlösung lyophilisiert. Man erhält 7,4 g eines farblosen Rückstands, der vorwiegend aus 7-(1-tert.-Butoxycarbonylaminomethyl-2-oxa-7-aza-bicyclo[3.3.0]oct-7-yl)-1 -cyclopropyl-6,8-difluor-1,4-dihydro-4-oxo-3-chinolincarbonsäure-Cäsiumsalz besteht, das durch etwas anorganisches Salz verunreinigt ist. - 637 mg diese Cäsium-Salzes werden in 5 ml Dimethylformamid bei 25 °C gelöst und mit 213 mg (1,5 mmol) Methyliodid versetzt. Man läßt über Nacht bei 25°C rühren, engt die Suspension am Rotationsverdampfer ein, behandelt den Rückstand mit Leichtbenzin und trocknet bei 80°C im Hochvakuum.
   Ausbeute: 360 mg 7-(1-tert.-Butoxycarbonylaminomethyl-2-oxa-7-aza-bicyclo[3.3.0]-oct-7-yl)-1-cyclopropyl-6,8-difluor-1,4-dihydro-4-oxo-3-chinolincarbonsäuremethylester,
   Schmelzpunkt: 141-144°C (unter Zersetzung).
B: Das aus Stufe A erhaltene Produkt wird in 25 ml Dichlormethan gelöst, mit 1,5 ml Trifluoressigsäure versetzt und während 2 Stunden bei Raumtemperatur gerührt. Die Lösung wird im Vakuum eingeengt, der Rückstand mit Dichlormethan/Methanol/17%-Ammoniak = 150:20:1) an Kieselgel chromatographisch gereinigt. Das erhaltene Produkt wird in 2 ml Wasser vorgelegt und mit 0,76 ml ln-HCl in Lösung gebracht. Man engt bei 60°C/15 mbar ein und trocknet das Salz bei 50°C im Hochvakuum.
   Ausbeute: 226 mg 7-(1-Aminomethyl-2-oxa-7-aza-bicyclo[3.3.0]oct-7-yl)-1-cyclopropyl-6,8-difluor-1,4-dihydro-4-oxo-3-chinolincarbonsäuremethylester-Hydrochlorid,
   Schmelzpunkt: 109-111°C.

### Beispiel 11

A: Analog Beispiel 10 A erhält man mit 1-Brombutan 7-(1-tert.-Butoxycarbonylaminomethyl-2-oxa-7-aza-bicyclo[3.3.0]oct-7-yl)-1-cyclopropyl-6,8-difluor-1,4-dihydro-4-oxo-3-chinolincarbonsäure(1-butyl)ester vom Schmelzpunkt 146-148°C.
B: Analog Beispiel 10 B wird mit Trifluoressigsäure die Schutzgruppe abgespalten und das erhaltene Trifluoracetat im ammoniakalischen Laufmittel chromatographisch gereinigt. Man isoliert 7-(1-Aminomethyl-2-oxa-7-aza-bicyclo[3.3.0]oct-7-yl)-1-cyclopropyl-6,8-difluor-1,4-dihydro-4-oxo-3-chinolincarbonsäure(1-butyl)ester vom Schmelzpunkt 117-119°C (unter Zersetzung).

### Beispiel 12

A: Analog Beispiel 10 A erhält man mit Allylbromid 7-(1-tert.-Butoxycarbonylaminomethyl-2-oxa-7-aza-bicyclo[3.3.0]oct-7-yl)-1-cyclopropyl-6,8-difluor-1,4-dihydro-4-oxo-3-chinolincarbonsäureallylester vom Schmelzpunkt 143-145°C.
B: Analog Beispiel 10 B wird mit Trifluoressigsäure die Schutzgruppe abgespalten und das erhaltene Trifluoracetat im ammoniakalischen Laufmittel chromatographisch gereinigt. Man isoliert 7-(1-Aminomethyl-2-oxa-7-aza-bicyclo[3.3.0]oct-7-yl)-1-cyclopropyl-6,8-difluor-1,4-dihydro-4-oxo-3-chinolincarbonsäureallylester vom Schmelzpunkt 133-135°C (unter Zersetzung).
C: Analog Beispiel 10 B wird mit Trifluoressigsäure die Schutzgruppe abgespalten und das erhaltene rohe Trifluoracetat mit Ethanol und Ether behandelt und als Feststoff isoliert: 7-(1-Aminomethyl-2-oxa-7-aza-bicyclo[3.3.0]oct-7-yl)-1-cyclopropyl-6,8-difluor-1,4-dihydro-4-oxo-3-chinolincarbonsäureallylester-Trifluoracetat vom Schmelzpunkt 118-119°C (unter Zersetzung).

### Beispiel 13

A: Analog Beispiel 10 A erhält man mit Propargylbromid 7-(1-tert.-Butoxycarbonylaminomethyl-2-oxa-7-aza-bicyclo[3.3.0]oct-7-yl)-1-cyclopropyl-6,8-difluor-1,4-dihydro-4-oxo-3-chinolincarbonsäurepropargylester vom Schmelzpunkt 159-160°C.
B: Analog Beispiel 10 B wird mit Trifluoressigsäure die Schutzgruppe abgespalten und das erhaltene Trifluoracetat im ammoniakalischen Laufmittel chromatographisch gereinigt. Man isoliert 7-(1-Aminomethyl-2-oxa-7-aza-bicyclo[3.3.0]oct-7-yl)-1-cyclopropyl-6,8-difluor-1,4-dihydro-4-oxo-3-chinolincarbonsäurepropargylester vom Schmelzpunkt 127-129°C (unter Zersetzung).

### Beispiel 14

A: Analog Beispiel 10 A erhält man mit Benzylylbromid 7-(1-tert.-Butoxycarbonylaminomethyl-2-oxa-7-aza-bicyclo[3.3.0]oct-7-yl)-1-cyclopropyl-6,8-difluor-1,4-dihydro-4-oxo-3-chinolincarbonsäurebenzylester vom Schmelzpunkt 148-149°C.
B: Analog Beispiel 10 B wird mit Trifluoressigsäure die Schutzgruppe abgespalten und das erhaltene Trifluoracetat im ammoniakalischen Laufmittel chromatographisch gereinigt. Man isoliert 7-(1-Aminomethyl-2-oxa-7-aza-bicyclo[3.3.0]oct-7-yl)-1-cyclopropyl-6,8-difluor-1,4-dihydro-4-oxo-3-chinolincarbonsäurebenzylester vom Schmelzpunkt 118-119°C (unter Zersetzung).
C: Analog Beispiel 10 B wird mit Trifluoressigsäure die Schutzgruppe abgespalten und das erhaltene rohe Trifluoracetat mit Ethanol und Ether behandelt und als Feststoff isoliert: 7-(1-Aminomethyl-2-oxa-7-aza-bicyclo[3.3.0]oct-7-yl)-1-cyclopropyl-6,8-difluor-1,4-dihydro-4-oxo-3-chinolincarbonsäurebenzylester-Trifluoracetat vom Schmelzpunkt 119-120°C (unter Zersetzung).

### Beispiel 15

A: Analog Beispiel 10 A erhält man mit Bromessigsäureethylester 7-(1-tert.-Butoxycarbonylaminomethyl-2-oxa-7-aza-bicyclo[3.3.0]oct-7-yl)-1-cyclopropyl-6,8-difluor-1,4-dihydro-4-oxo-3-chinolincarbonsäure(ethoxycarbonylmethyl)ester vom Schmelzpunkt 150-152°C.
B: Analog Beispiel 10 B wird mit Trifluoressigsäure die Schutzgruppe abgespalten und das erhaltene Trifluoracetat im ammoniakalischen Laufmittel chromatographisch gereinigt. Man isoliert 7-(1-Aminomethyl-2-oxa-7-aza-bicyclo[3.3.0]oct-7-yl)-1-cyclopropyl-6,8-difluor-1,4-dihydro-4-oxo-3-chinolincarbonsaure(ethoxycarbonylmethyl)ester vom Schmelzpunkt 144-146°C (unter Zersetzung).

### Beispiel 16

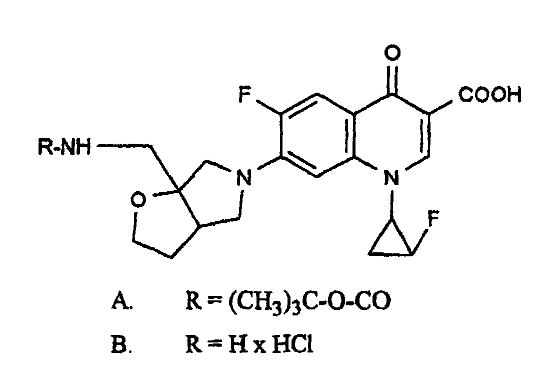

A: Analog Beispiel 1A setzt man mit 1-(cis-2-Fluor-cyclopropyl)-6,7-difluor-1,4-dihydro-4-oxo-3-chinolincarbonsäure zu 7-(1-tert.-Butoxycarbonylaminomethyl-2-oxa-7-aza-bicyclo[3.3.0]oct7-yl)-1-(cis-2-fluor-cyclopropyl)-6-fluor-1,4-dihydro-4-oxo-3-chinolincarbonsäure (74 % der Theorie) vom Schmelzpunkt 202-206°C (unter Zersetzung) um.
B: Analog Beispiel 1B wird das Produkt aus der Stufe A mit halbkonzentrierter Salzsäure zu 7-(1-Aminomethyl-2-oxa-7-aza-bicyclo[3.3.0]oct-7-yl)-1-(cis-2-fluor-cyclopropyl)-6-fluor-1,4-dihydro-4-oxo-3-chinolincarbonsäure-Hydrochlorid vom Schmelzpunkt 257-259°C (unter Zersetzung) umgesetzt.

### Beispiel 17

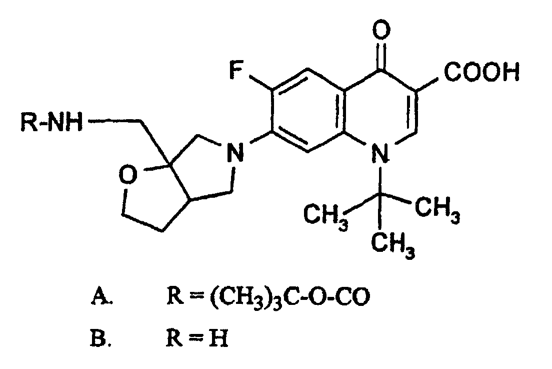

A: Analog Beispiel 1A setzt man mit l-tert.-Butyl-6,7-difluor-1,4-dihydro-4-oxo-3-chinolincarbonsäure zu 7-(1-tert.-Butoxycarbonylaminomethyl-2-oxa-7-aza-bicyclo[3.3.0]oct7-yl)-1-tert.-butyl-6-fluor-1,4-dihydro-4-oxo-3-chinolincarbonsäure (84 % der Theorie) vom Schmelzpunkt 131-133°C (unter Zersetzung) um.
B: Analog Beispiel 1B wird das Produkt aus der Stufe A mit halbkonzentrierter Salzsäure während 15 Minuten deblockiert und das erhaltene Rohprodukt in einem ammoniakalischen Laufmittel (Dichlormethan/Methanol/17 %-Ammoniak 30:8:1) an Kieselgel chrromatographisch gereinigt. Man erhält neben 7-(1-Aminomethyl-2-oxa-7-aza-bicyclo[3.3.0]oct-7-yl)-6-fluor-1,4-dihydro-4-oxo-3-chinolincarbonsäure (Schmelzpunkt: >330°C) 7-(1-Aminomethyl-2-oxa-7-aza-bicyclo[3.3.0]oct-7-yl)-1-tert.-butyl-6-fluor-1,4-dihydro-4-oxo-3-chinolincarbonsäure vom Schmelzpunkt 217-218°C (unter Zersetzung).

### Beispiel 18

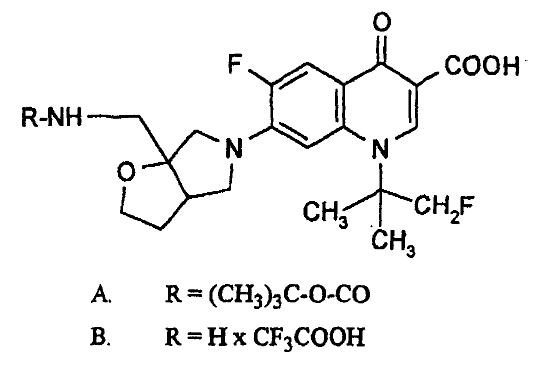

A: Analog Beispiel 1A setzt man mit 1-(Fluor-tert.-butyl)-6,7-difluor-1,4-dihydro-4-oxo-3-chinolincarbonsäure zu 7-(1-tert.-Butoxycarbonylaminomethyl-2-oxa-7-azabicyclo[3.3.0]oct7-yl)-1-(fluor-tert.-butyl)-6-fluor-1,4-dihydro-4-oxo-3-chinolincarbonsäure (81 % der Theorie) vom Schmelzpunkt 161-162°C (unter Zersetzung) um.
B: Analog Beispiel 1B wird das Produkt aus der Stufe A mit Trifluoressigsäure deblockiert und das nach dem Einengen erhaltene Rohprodukt durch Behandeln mit Ethanol zur Kristallisation gebracht. Man erhält 7-(1-Aminomethyl-2-oxa-7-aza-bicyclo[3.3.0]oct-7-yl)-1-(fluor-tert.-butyl)-6-fluor-1,4-dihydro-4-oxo-3-chinolincarbonsäure-Trifluoracetat vom Schmelzpunkt 197-198°C (unter Zersetzung).

### Beispiel 19

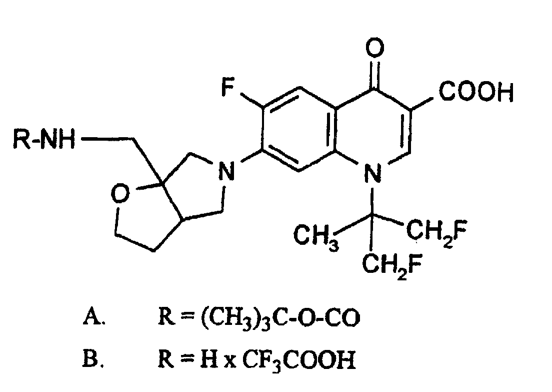

A: Analog Beispiel 1A setzt man mit 1-(Difluor-tert.-butyl)-6,7-difluor-1,4-dihydro-4-oxo-3-chinolincarbonsäure zu 7-(1-tert.-Butoxycarbonylaminomethyl-2-oxa-7-azabicyclo[3.3.0]oct7-yl)-1-(difluor-tert.-butyl)-6-fluor-1,4-dihydro-4-oxo-3-chinolincarbonsäure (59 % der Theorie) um.
B: Analog Beispiel 6C wird das Produkt aus der Stufe A mit Trifluoressigsäure in Dichlormethan deblockiert, das nach dem Einengen erhaltene Rohprodukt in Wasser aufgenommen, mit Dichlormethan gewaschen und die wäßrige Phase lyophilisiert. Man erhält in 12 %-iger Ausbeute 7-(1-Aminomethyl-2-oxa-7-aza-bicyclo[3.3.0]oct-7-yl)-1-(difluor-tert.-butyl)-6-fluor-1,4-dihydro-4-oxo-3-chinolincarbonsäure-Trifluoracetat vom Schmelzpunkt 154-160°C (unter Zersetzung).

### Beispiel 20

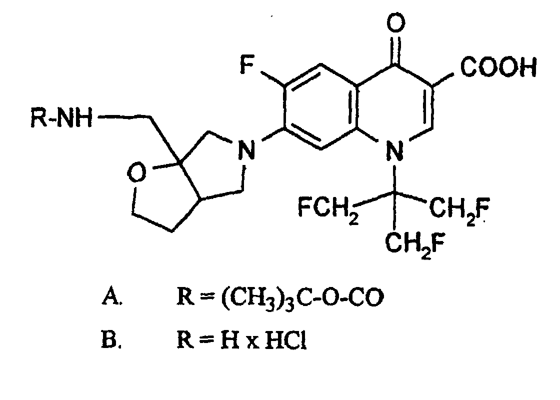

A: Analog Beispiel 1A setzt man mit l-(Trifluor-tert.-butyl)-6,7-difluor-1,4-dihydro-4-oxo-3-chinolincarbonsäure zu 7-(1-tert.-Butoxycarbonylaminomethyl-2-oxa-7-azabicyclo[3.3.0]oct7-yl)-1-(trifluor-tert.-butyl)-6-fluor-1,4-dihydro-4-oxo-3-chinolincarbonsäure (73 % der Theorie) vom Schmelzpunkt 131°C um.
B: Analog Beispiel 1B wird das Produkt aus der Stufe A mit Salzsäure deblockiert. Man erhält in 41 %-iger Ausbeute 7-(1-Aminomethyl-2-oxa-7-aza-bicyclo[3.3.0]oct-7-yl)-1-(trifluor-tert.-butyl)-6-fluor-1,4-dihydro-4-oxo-3-chinolincarbonsäure-Hydrochlorid vom Schmelzpunkt 228°C (unter Zersetzung).

### Beispiel 21

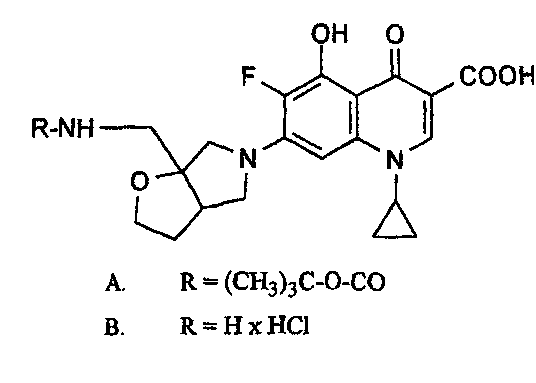

A: Analog Beispiel 1A setzt man mit 1-Cyclopropyl-6,7-difluor-5-hydroxy-1,4-dihydro-4-oxo-3-chinolincarbonsäure zu 7-(1-tert.-Butoxycarbonylaminomethyl-2-oxa-7-aza-bicyclo[3.3.0]oct-7-yl)-1-cyclopropyl-6-fluor-5-hydroxy-1,4-dihydro-4-oxo-3-chinolincarbonsäure ( 63 % der Theorie) um.
B: Analog Beispiel 1B wird das Produkt aus der Stufe A mit halbkonzentrierter Salzsäure zu 7-(1-Aminomethyl-2-oxa-7-aza-bicyclo[3.3.0]oct-7-yl)-1-cyclopropyl-6-fluor-5-hydroxy-1,4-dihydro-4-oxo-3-chinolincarbonsäure-Hydrochlorid vom Schmelzpunkt 225°C (unter Zersetzung) umgesetzt.

### Beispiel 22

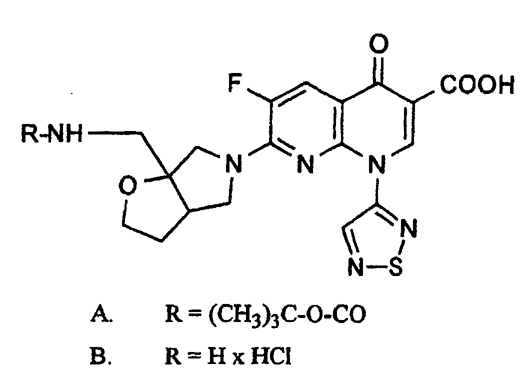

A: Analog Beispiel 1A setzt man mit 7-Chlor-6-fluor-1,4-dihydro-4-oxo-1-(1,2,5-thiadiazol-3-yl)-1,8-naphthyridin-3-carbonsäure zu 7-(1-tert.-Butoxycarbonylaminomethyl-2-oxa-7-aza-bicyclo[3.3.0]oct-7-yl)-6-fluor-1,4-dihydro-4-oxo-1-(1,2,5-thiadiazol-3-yl)-1,8-naphthyridin-3-carbonsäure (66 % der Theorie) um.
B: Analog Beispiel 1B wird das Produkt aus der Stufe A mit halbkonzentrierter Salzsäure zu 7-(1-Aminomethyl-2-oxa-7-aza-bicyclo[3.3.0]oct-7-yl)-6-fluor-1,4-dihydro-4-oxo-1-(1,2,5-thiadiazol-3-yl)-1,8-naphthyridin-3-carbonsäure-Hydrochlorid vom Schmelzpunkt 207°C (unter Zersetzung) umgesetzt.

### Beispiel 23

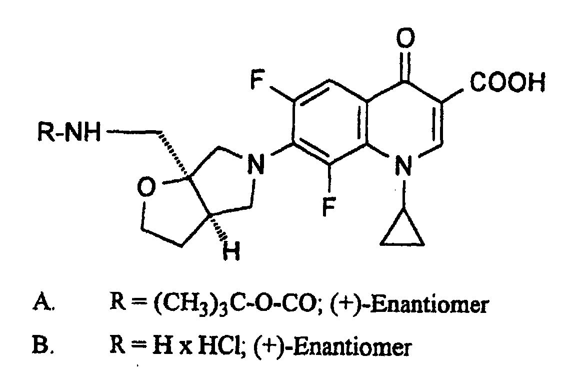

A: Eine Mischung von 481 mg (1,7 mmol) 1-Cyclopropyl-6,7,8-trifluor-1,4-dihydro-4-oxo-3-chinolincarbonsäure in 5 ml Acetonitril und 2,5 ml Dimethylformamid mit 217 mg (1,9 mmol) 1,4-Diazabicyclo[2.2.2]octan und 467 mg (1,9 mmol) (+)-(1R,5R)-1-tert.-Butoxycarbonylaminomethyl-2-oxa-7-aza-bicyclo[3.3.0]octan wird 5 Stunden unter Rückfluß erhitzt und die Lösung heiß filtriert. Es kristallisiert das Reaktionsprodukt aus, das abgesaugt und mit wenig Acetonitril gewaschen wird. Der Niederschlag wird anschließend mit ca. 40 ml Wasser gut verrührt, abgesaugt und bei 80°C im Hochvakuum getrocknet.
   Ausbeute: 748 mg (87 % der Theorie) (+)-(1R,5R)-7-(1-tert.-Butoxycarbonylaminomethyl-2-oxa-7-aza-bicyclo[3.3.0]oct-7-yl)-1-cyclopropyl-6,8-difluor-1,4-dihydro-4-oxo-3 -chinolincarbonsäure,
   Schmelzpunkt: 202-203°C (unter Zersetzung).
   [α]³ : +94° (c = 0,46, CHCl₃).
B: 701 mg (1,39 mmol) (+)-(1R,5R)-7-(1-tert.-Butoxycarbonylaminomethyl-2-oxa-7-aza-bicyclo[3.3.0]oct-7-yl)-1-cyclopropyl-6,8-difluor-1,4-dihydro-4-oxo-3-chinolincarbonsäure werden in 16 ml halbkonzentrierter Salzsäure in der Wärme gelöst, die Lösung filtriert und bei 60 C/15 mbar eingeengt. Der Rückstand wird mit etwas Ethanol versetzt, erneut eingeengt, das Salz isoliert und bei 80 C im Hochvakuum getrocknet.
   Ausbeute: 537 mg (88 % der Theorie) (+)-(1S,5R)-7-(Aminomethyl-2-oxa-7-azabicyclo[3.3.0]oct-7-yl)-1-cyclopropyl-6,8-difluor-1,4-dihydro-4-oxo-3-chinolincarbonsäure-Hydrochlorid,
   Schmelzpunkt: 214-216°C (unter Zersetzung).
   [α]³ : +49° (c = 0,45, DMF).
   ee > 99,8 % (bestimmt durch Kapillarelektrophorese).

### Beispiel 24

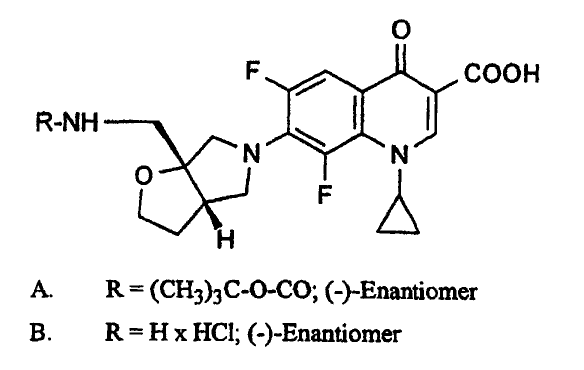

A: Analog Beispiel 23 A erhält man mit (-)-(1S,5S)-1-tert.-Butoxycarbonylaminomethyl-2-oxa-7-aza-bicyclo[3.3.0]octan in 67 % Ausbeute (-)-(1S,5S)-7-(1-tert.-Butoxycarbonylaminomethyl-2-oxa-7-aza-bicyclo[3.3.0]oct-7-yl)-1-cyclopropyl-6,8-difluor-1,4-dihydro-4-oxo-3-chinolincarbonsäure,
   Schmelzpunkt: 209-210°C (unter Zersetzung).
   [α]³ : -96° (c = 0,43, CHCl₃).
B: Analog Beispiel 23 B wird das Produkt aus Stufe A durch Umsetzung mit Salzsäure zu (-)-(1R,5S)-7-(Aminomethyl-2-oxa-7-aza-bicyclo[3.3.0]oct-7-yl)-1-cyclopropyl-6,8-difluor-1,4-dihydro-4-oxo-3-chinolincarbonsäure-Hydrochlorid deblockiert,
   Schmelzpunkt: 214-216°C (unter Zersetzung).
   [α]³ : -49° (c = 0,33, DMF).
   ee > 99,8 % (bestimmt durch Kapillarelektrophorese).

### Beispiel 25

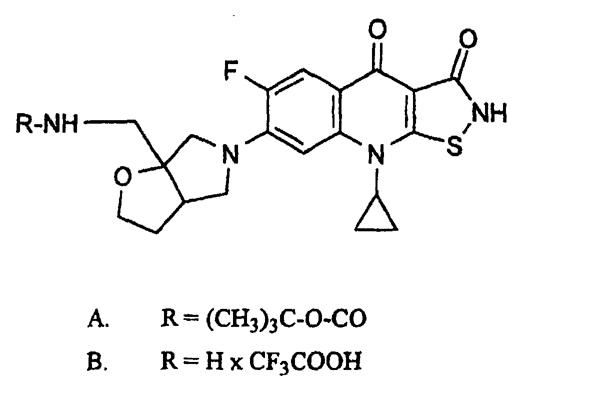

A: 250 mg (0,85 mmol) 9-Cyclopropyl-6,7-difluor-2,3,4,9-tetrahydroisothiazolo[5,4-b]chinolin-3,4-dion werden in 10 ml absolutem Pyridin mit 247 mg (1,02 mmol) 1-tert.-Butoxycarbonylaminomethyl-2-oxa-7-aza-bicyclo[3.3.0]octan über Nacht unter Rückfluß erhitzt. Nach dem Abkühlen wird mit 40 ml Wasser versetzt, der ausgefallene Niederschlag abgesaugt, mit Wasser gewaschen und im Vakuum getrocknet. Man erhält 190 mg 7-(1-tert.-Butoxycarbonylaminomethyl-2-oxa-7-aza-bicyclo[3.3.0]oct-7-yl)-9-cylopropyl-6-fluor-2,3,4,9-tetrahydroisothiazolo[5,4-b]chinolin-3,4-dion.
B: Das Produkt aus Stufe A wird analog Beispiel 6C mit Trifluoressigsäure in Dichlormethan deblockiert, die Lösung eingeengt, mehrmals mit etwas Toluol abgedampft, der Rückstand in 5 ml Methanol aufgenommen und das Salz mit 20 ml Isopropanol ausgefällt.
Ausbeute: 163 mg 7-(1-Aminomethyl-2-oxa-7-aza-bicyclo[3.3.0]oct-7-yl)-9-cylopropyl-6-fluor-2,3,4,9-tetrahydroisothiazolo[5,4-b]chinolin-3,4-dion-Trifluoracetat,
   Schmelzpunkt: 215°C (unter Zersetzung).

### Beispiel 26

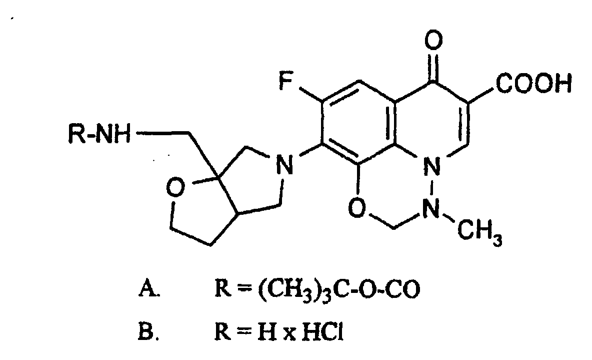

A: 0,72 g (2,72 mmol) 9,10-Difluor-3-methyl-7-oxo-2,3-dihydro-7H-pyrido[1,2,3-de][1,3,4]-benzoxdiazin-6-carbonsäure werden in 36 ml Acetonitril mit 0,4 g (2,8 mmol) 1-tert.-Butoxycarbonylaminomethyl-2-oxa-7-aza-bicyclo[3.3.0]octan und 0,6 g 1,4-Diazabicyclo[2.2.2]octan versetzt und 16 Stunden unter Rückfluß erhitzt. Man engt ein, nimmt in Wasser auf und extrahiert mit Dichlormethan. Dann trocknet man mit Natriumsulfat, engt ein, verrührt mit Methanol, isoliert den erhaltenen Feststoff und trocknet im Vakuum. Man erhält 0,6 g 10-(1-tert.-Butoxycarbonylaminomethyl-2-oxa-7-aza-bicyclo[3.3.0]oct-7-yl)-9-fluor-3-methyl-7-oxo-2,3-dihydro-7H-pyrido[1,2,3-de][1,3,4]-benzoxdiazin-6-carbonsäure vom Schmelzpunkt 176°C.
B: 0,6 g (1,9 mmol) des Produkts aus Stufe A werden in 105 ml 4N Salzsäure/Dioxan (1:1) gelöst und 2 Stunden auf 60°C erwärmt. Die Lösung wird eingeengt, mit etwas Ethanol verrührt, der Niederschlag abgesaugt und getrocknet.
   Ausbeute: 110 mg 10-(1-Aminomethyl-2-oxa-7-aza-bicyclo[3.3.0]oct-7-yl)-9-fluor-3-methyl-7-oxo-2,3-dihydro-7H-pyrido[1,2,3-de][1,3,4]-benzoxdiazin-6-carbonsäure-Hydrochlorid,
   Schmelzpunkt: 233°C.

### Beispiel 27

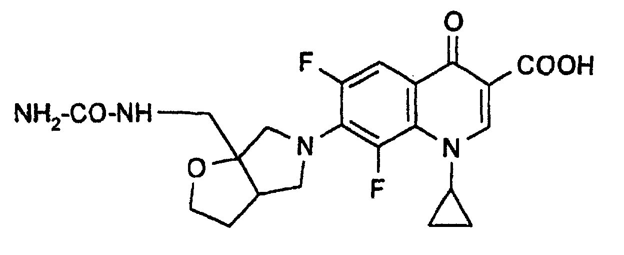

Eine Mischung von 283 mg (1 mmol) 1-Cyclopropyl-6,7,8-trifluor-1,4-dihydro-4-oxo-3-chinolincarbonsäure in 4 ml Acetonitril und 2 ml Dimethylformamid mit 112 mg (1 mmol) 1,4-Diazabicyclo[2.2.2]octan und 234 mg (1,3 mmol) 1-Ureidomethyl-2-oxa-7-aza-bicyclo[3.3.0]octan wird 2 Stunden unter Rückfluß erhitzt. Die Mischung wird eingeengt, der Rückstand mit etwas Wasser versetzt und im Ultraschallbad behandelt. Der ungelöste Niederschlag wird abgesaugt, mit Wasser gewaschen und bei 100°C im Hochvakuum getrocknet.

Ausbeute: 237 mg (53 % der Theorie) 1-Cyclopropyl-6,8-difluor-1,4-dihydro-4-oxo-7-(1-ureidomethyl-2-oxa-7-aza-bicyclo[3.3.0]oct-7-yl)-3-chinolincarbonsäure,
Schmelzpunkt: 185-187°C (unter Zersetzung).

### Beispiel 28

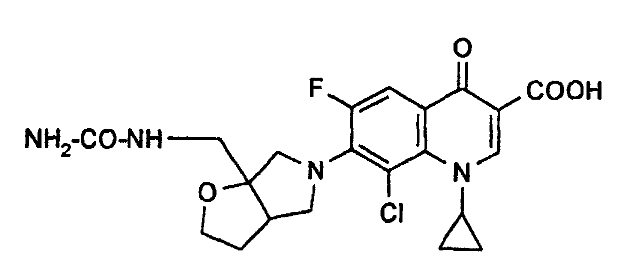

Analog Beispiel 27 erhält man 8-Chlor-1-cyclopropyl-6-fluor-1,4-dihydro-4-oxo-7-(1-ureidomethyl-2-oxa-7-aza-bicyclo[3.3.0]oct-7-yl)-3-chinolincarbonsäure,
Schmelzpunkt: 232-234°C (unter Zersetzung).

### Beispiel 29

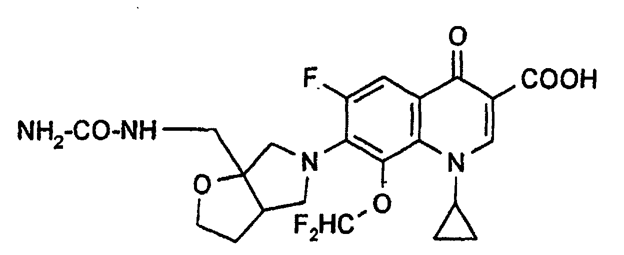

Analog Beispiel 27 erhält man 1-Cyclopropyl-8-difluormethoxy-6-fluor-1,4-dihydro-4-oxo-7-(1-ureidomethyl-2-oxa-7-aza-bicyclo[3.3.0]oct-7-yl)-3-chinolincarbonsäure,
Schmelzpunkt: 232-234°C (unter Zersetzung).

### Beispiel 30

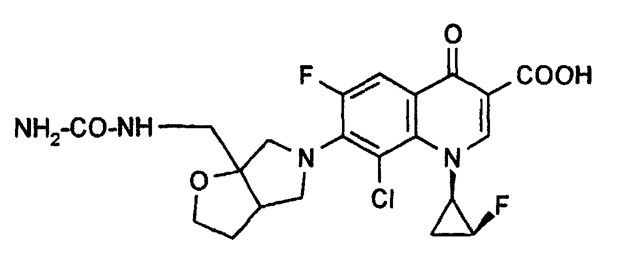

Analog Beispiel 27 erhält man 8-Chlor-6-fluor-1-[(1R,2S)-2-fluor-cyclopropyl]-1,4-dihydro-4-oxo-7-(1-ureidomethyl-2-oxa-7-aza-bicyclo[3.3.0]oct-7-yl)-3-chinolincarbonsäure,
Schmelzpunkt: 208-210°C (unter Zersetzung).

### Beispiel 31

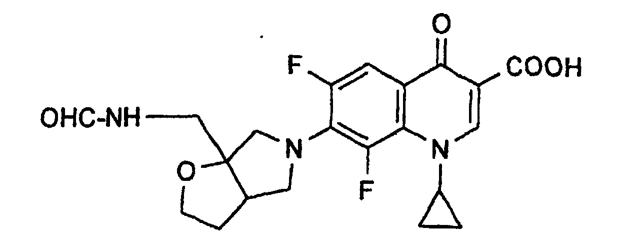

Man erhitzt 310 mg (1 mmol) 7-(1-Aminomethyl-2-oxa-7-aza-bicyclo[3.3.0]oct-7-yl)-1-cyclopropyl-6,8-difluor-1,4-dihydro-4-oxo-3-chinolincarbonsäure in 2,3 ml Dimethylformamid mit 2,3 ml Ameisensäure 8 Stunden unter Rückfluß. Die Mischung wird im Vakuum eingeengt, der Rückstand mit 8 ml Wasser verrührt, der ausgefallene Niederschlag abgesaugt, getrocknet und mit Dichlormethan/Methanol (95:5) als Laufmittel an Kieselgel chromatographiert.

Ausbeute: 173 mg (40 % der Theorie) 1-Cyclopropyl-6,8-difluor-7-(1-formylaminomethyl-2-oxa-7-aza-bicyclo[3.3.0]oct-7-yl)-1,4-dihydro-4-oxo-3-chinolincarbonsäure,
Schmelzpunkt: 208-209°C (unter Zersetzung).

### Beispiel 32

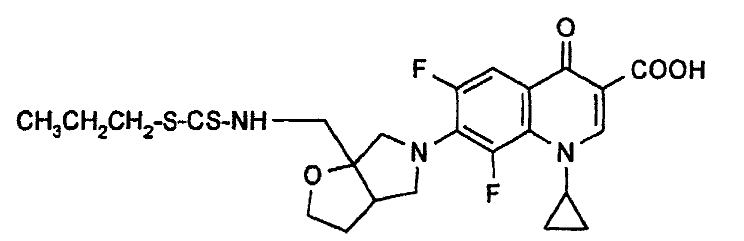

730 mg (2 mmol) 7-(1-Aminomethyl-2-oxa-7-aza-bicyclo[3.3.0]oct-7-yl)-1-cyclopropyl-6,8-difluor-1,4-dihydro-4-oxo-3-chinolincarbonsäure werden in einer Mischung aus 1,3 ml Wasser und 1 ml 4n-Natronlauge bei Raumtemperatur vorgelegt, die Mischung mit Eis gekühlt und mit 0,24 ml Schwefelkohlenstoff versetzt. Man rührt 1 Stunde bei 5°C und anschließend noch 15 Stunden bei Raumtemperatur. Die Suspension wird mit 50 ml Aceton versetzt, gekühlt und der Niederschlag abgesaugt, mit Aceton gewaschen und bei 80°C im Hochvakuum getrocknet. Man erhält 657 mg des Natriumsalzes des Dithiourethans. 409 mg dieses Natriumsalzes werden in 5 ml Dimethylformamid vorgelegt und mit 123 mg 1-Brompropan in 1 ml Dimethylformamid versetzt und die Mischung über Nacht bei Raumtemperatur gerührt. Man engt bei 70°C/12 mbar ein, verrührt den Rückstand mit Ethanol, saugt den Niederschlag, tocknet und reinigt das erhaltene Rohprodukt (225 mg) durch Chromatographie an Kieselgel mit Dichlormethan/Methanol/17 %-Ammoniak (150:4:1).

Ausbeute: 56 mg 7-(1-Propylthio-thiocarbonylaminomethyl-2-oxa-7-aza-bicyclo[3.3.0]oct-7-yl)-1-cyclopropyl-6,8-difluor-1,4-dihydro-4-oxo-3-chinolincarbonsäure,
Schmelzpunkt: 162-166°C (unter Zersetzung).

### Beispiel 33

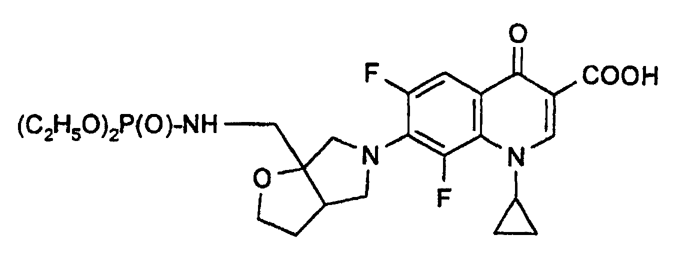

395 mg (1 mmol) 7-(1-Aminomethyl-2-oxa-7-aza-bicyclo[3.3.0]oct-7-yl)-1-cyclopropyl-6,8-difluor-1,4-dihydro-4-oxo-3-chinolincarbonsäure werden in 15 ml Dichlormethan mit 406 mg (2 mmol) Bis(trimethylsilyl)acetamid versetzt und 3 Stunden bei 25°C gerührt. Anschließend wird mit 173 mg (1 mmol) Diethylphosphorylchlorid versetzt und 24 Stunden bei Raumtemperatur gerührt. Es wird von unverändertem Edukt abgesaugt, die Mutterlauge eingeengt und der Rückstand an Kieselgel chromatographiert (Laufmittel: Dichlormethan/Methanol 95:5).

Ausbeute: 73 mg (1,5 %) 7-(1-Diethoxyphosphoryl-aminomethyl-2-oxa-7-aza-bicyclo[3.3.0]oct-7-yl)-1-cyclopropyl-6,8-difluor-1,4-dihydro-4-oxo-3-chinolincarbonsäure,
FAB-MS: m/e 542 [(M+H)⁺], 524.

### Beispiel 34

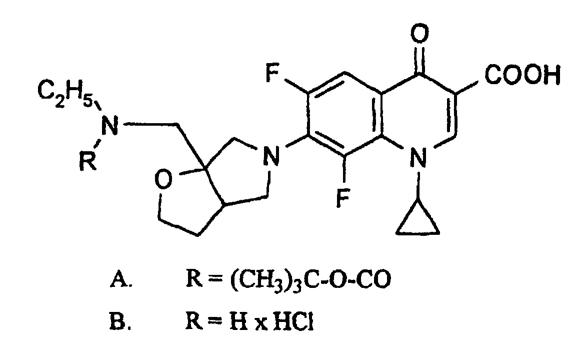

A: Eine Mischung von 283 mg (1 mmol) 1-Cyclopropyl-6,7,8-trifluor-1,4-dihydro-4-oxo-3-chinolincarbonsäure in 3 ml Acetonitril und 1,5 ml Dimethylformamid mit 120 mg (1,07 mmol) 1,4-Diazabicyclo[2.2.2]octan und 400 mg (1,5 mmol) 1-(N-tert.-Butoxycarbonyl-N-ethyl-aminomethyl)-2-oxa-7-aza-bicyclo[3.3.0]octan wird 6 Stunden unter Rückfluß erhitzt und die Lösung eingegengt, mit 20 ml Wasser versetzt und im Ultraschallbad behandelt. Der ungelöste Niederschlag wird abgesaugt, mit Wasser gewaschen und bei 80°C im Hochvakuum getrocknet.
   Ausbeute: 460 mg (86 % der Theorie) 7-[1-(N-tert.-Butoxycarbonyl-N-ethyl-aminomethyl)-2-oxa-7-aza-bicyclo[3.3.0]oct-7-yl]-1-cyclopropyl-6,8-difluor-1,4-dihydro-4-oxo-3-chinolincarbonsäure,
   Schmelzpunkt: 166-168°C (unter Zersetzung).
B: 430 mg (0,8 mmol) des Produkts aus Stufe A werden in 15 ml halbkonzentrierter Salzsäure in der Wärme gelöst, die Lösung filtriert und bei 60 C/15 mbar eingeengt. Der Rückstand wird mit etwas Ethanol versetzt, erneut eingeengt, das Salz isoliert und bei 80 C im Hochvakuum getrocknet.
   Ausbeute: 272 mg (72 % der Theorie) 7-(Ethylaminomethyl-2-oxa-7-aza-bicyclo[3.3.0]oct-7-yl)-1-cyclopropyl-6,8-difluor-1,4-dihydro-4-oxo-3-chinolincarbonsäure-Hydrochlorid,
   Schmelzpunkt: 280-281°C (unter Zersetzung).

### Beispiel 35

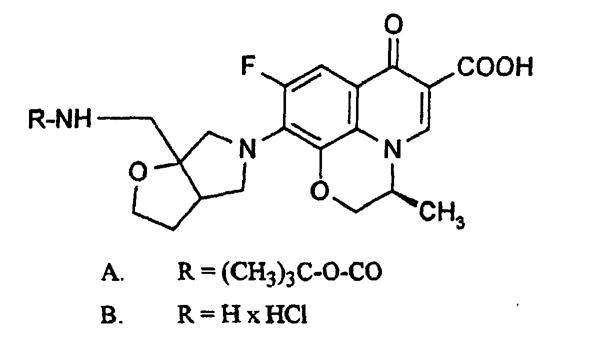

A: Analog Beispiel 1A setzt man mit 9,10-Difluor-3(S)-methyl-7-oxo-2,3-dihydro-7H-pyrido[1,2,3-de][1,4]benzoxacin-6-carbonsäure zu 10-(1-tert.-Butoxycarbonylaminomethyl-2-oxa-7-aza-bicyclo[3.3.0]oct-7-yl)-9-fluor-3(S)-methyl-7-oxo-2,3-dihydro-7H-pyrido[1,2,3-de][1,4]benzoxacin-6-carbonsäure vom Schmelzpunkt 157-158°C (unter Zersetzung) um.
B: Analog Beispiel 1B wird das Produkt aus der Stufe A mit halbkonzentrierter Salzsäure zu 10-(1-Aminomethyl-2-oxa-7-aza-bicyclo[3.3.0]oct-7-yl)-9-fluor-3(S)-methyl-7-oxo-2,3-dihydro-7H-pyrido[1,2,3-de][1,4]benzoxacin-6-carbonsäure-Hydrochlorid vom Schmelzpunkt 191-193°C (unter Zersetzung) umgesetzt.

### Beispiel 36

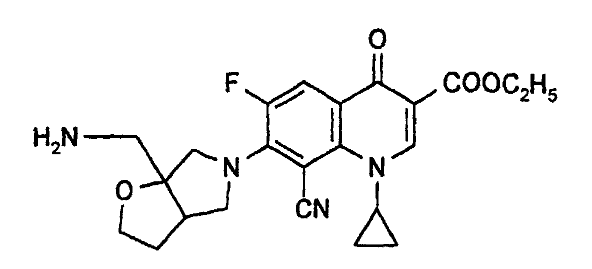

350 mg (1,21 mmol) 8-Cyano-1-cyclopropyl-6,7-difluor-1,4-dihydro-4-oxo-chinolincarbonsäureethylester und 260 mg (1,2 mmol) (1SR,5RS)-1-Aminomethyl-2-oxa-7-azabicyclo[3.3.0]octan-Dihydrochlorid werden in 30 ml absolutem Acetonitril mit 470 mg (4,64 mmol) Triethylamin versetzt und über Nacht bei Raumtemperatur gerührt. Die Mischung wird eingeengt und der Rückstand chromatographisch (Kieselgel; Dichlormethan/Methanol 9:1) gereinigt. Man isoliert 450 mg (86 % der Theorie) 7-([1SR,5RS]-1-Aminomethyl-2-oxa-7-azabicyclo[3.3.0]oct-7-yl)-8-cyano-1-cyclopropyl-6-fluor-1,4-dihydro-4-oxo-chinolincarbonsäureethylester, Schmelzpunkt: 193°C.

Entsprechend werden auch 7-([1R,5S]-1-Aminomethyl-2-oxa-7-azabicyclo[3.3.0]oct-7-yl)-8-cyano-1-cyclopropyl-6-fluor-1,4-dihydro-4-oxo-chinolincarbonsäureethylester und 7-([1S,5R]-1-Aminomethyl-2-oxa-7-azabicyclo[3.3.0]oct-7-yl)-8-cyano-1-cyclopropyl-6-fluor-1,4-dihydro-4-oxo-chinolincarbonsäureethylester hergestellt.

## Patentansprüche

1. Verwendung von Verbindungen der Formel (I) 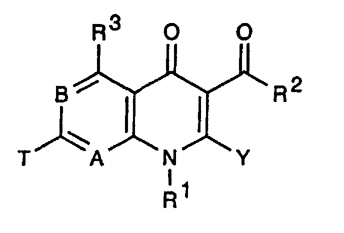 worin
R¹ für gegebenenfalls durch Halogen oder Hydroxy ein- bis dreifach substituiertes Alkyl mit 1 bis 4 Kohlenstoffatomen, Alkenyl mit 2 bis 4 Kohlenstoffatomen, gegebenenfalls durch 1 oder 2 Fluoratome substituiertes Cycloalkyl mit 3 bis 6 Kohfenstoffatomen, Bicyclo[1.1.1]pent-1-yl, 1,1-Dimethylpropargyl, 3-Oxetanyl, Methoxy, Amino, Methylamino, Dimethylamino, gegebenenfalls durch Halogen, Amino oder Hydroxy ein- oder zweifach substituiertes Phenyl, Isoxazolyl, Thiadiazolyl,
R² für Hydroxy, gegebenenfalls durch Hydroxy, Methoxy, Amino, Dimethylamino oder Ethoxycarbonyl substituiertes Alkoxy mit 1 bis 4 Kohlenstoffatomen, Benzyloxy, Allyloxy, Propargyloxy oder (5-Methyl-2-oxo-1,3-dioxol-4-yl)-methyloxy, Acetoxymethyloxy, Pivaloyloxymethyloxy, 5-Indanyloxy, Phthalidinyloxy, 3-Acetoxy-2-oxo-butyloxy, Nitromethyl oder Dialkoxycarbonylmethyl mit 1 bis 2 Kohlenstoffatomen in jedem Alkylteil,
R³ für Wasserstoff, Amino, Hydroxy, Methyl oder Halogen,
A für N oder C-R⁷ steht, worin
R⁷ für Wasserstoff, Halogen, CF₃, OCH₃, OCHF₂, CH₃, CN, CH=CH₂ oder C CH steht oder auch gemeinsam mit R¹ eine Brücke der Struktur -*O-CH₂-CH-CH₃, -*S-CH₂-CH₂-, -*S-CH₂-CH-CH₃, -*CH₂-CH₂-CH-CH₃ oder -*O-CH₂-N-R⁸, wobei das mit * markierte Atom mit dem Kohlenstoffatom von A verknüpft ist und worin
R⁸ Wasserstoff, Methyl oder Formyl bedeutet,
bilden kann,
B für N, C-H, C-F, C-Cl, C-NO₂, C-NH₂,
Y für Wasserstoff steht oder gemeinsam mit R² eine Brücke der Struktur -*S-NH- bilden kann, wobei das mit * markierte Atom für Y steht, und
T einen Rest der Formel 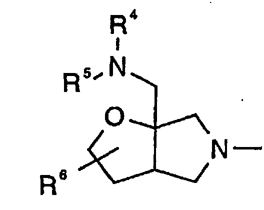 bedeutet, worin
R⁴ für H, CH₃, C₂H₅, gegebenenfalls durch Amino substituiertes Acyl mit 1 bis 5 Kohlenstoffatomen, Alkoxycarbonyl, Aminocarbonyl, Alkylthio-thiocarbonyl und Dialkoxyphosphoryl mit 1 bis 4 Kohlenstoffatomen im Alkylteil,
R⁵ für H, CH₃, C₂H₅ und
R⁶ für H, CH₃ stehen,
und deren pharmazeutisch verwendbaren Hydrate und Säureadditionssalze sowie die Alkali-, Erdalkali-, Silber- und Guanidiniumsalze der zugrundeliegenden Carbonsäuren zur Herstellung von Arzneimitteln für die Therapie von Helicobacter-pylori-Infektionen und den damit assoziierten gastroduodenalen Erkrankungen.

2. Verwendung gemäß Anspruch 1, wobei in Formel (I)
R¹ für gegebenenfalls durch Fluor ein- bis dreifach substituiertes Alkyl mit 1 bis 4 Kohlenstoffatomen, Vinyl, gegebenenfalls durch Fluor substituiertes Cyclopropyl, Bicyclo[1.1.1]pent-1-yl, 1,1-Dimethylpropargyl, 3-Oxetanyl, Methylamino, gegebenenfalls durch Fluor, Amino oder Hydroxy ein- oder zweifach substituiertes Phenyl, Thiadiazolyl,
R² für Hydroxy, gegebenenfalls durch Ethoxycarbonyl substituiertes Alkoxy mit 1 bis 4 Kohlenstoffatomen, Benzyloxy, Allyloxy, Propargyloxy,
R³ für Wasserstoff, Amino, Hydroxy, Methyl oder Fluor,
A für N oder C-R⁷ steht, worin
R⁷ für Wasserstoff, Halogen, CF₃, OCH₃, OCHF₂, CH₃, CN, CH=CH₂ oder C CH steht oder auch gemeinsam mit R¹ eine Brücke der Struktur -*O-CH₂-CH-CH₃ oder -*O-CH₂-N-R⁸, wobei das mit *markierte Atom mit dem Kohlenstoffatom von A verknüpft ist und worin
R⁸ Wasserstoff oder Methyl bedeutet,
bilden kann,
B für N, C-H, C-F, C-Cl, GNH₂,
Y für Wasserstoff steht oder gemeinsam mit R² eine Brücke der Struktur -*S-NH- bilden kann, wobei das mit * markierte Atom für Y steht, und
T einen Rest der Formel 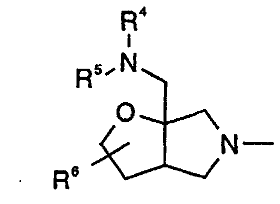 bedeutet, worin
R⁴ für H, CH₃, C₂H₅, gegebenenfalls durch Amino substituiertes Acyl mit 1 bis 5 Kohlenstoffatomen, Alkoxycarbonyl, Aminocarbonyl, Alkylthio-thiocarbonyl und Dialkoxyphosphoryl mit 1 bis 4 Kohlenstoffatomen im Alkylteil,
R⁵ für H, CH₃, C₂H₅ und
R⁶ für H stehen.

3. Verwendung gemäß Anspruch 1, wobei in Formel (I)
R¹ für gegebenenfalls durch Fluor ein- oder zweifach substituiertes Alkyl mit 1 bis 4 Kohlenstoffatomen, gegebenenfalls durch Fluor substituiertes Cyclopropyl, gegebenenfalls durch Fluor ein- oder zweifach substituiertes Phenyl,
R² für Hydroxy, gegebenenfalls durch Ethoxycarbonyl substituiertes Alkoxy mit 1 bis 4 Kohlenstoffatomen, Benzyloxy, Allyloxy, Propargyloxy,
R³ für Wasserstoff, Amino, Hydroxy, Methyl oder Fluor,
A für N oder C-R⁷ steht, worin
R⁷ für Wasserstoff, Chlor, Fluor, OCH₃, OCHF₂, CH₃ oder CN steht oder auch gemeinsam mit R¹ eine Brücke der Struktur -*O-CH₂-CH-CH₃ oder -*O-CH₂-N-CH₃, wobei das mit * markierte Atom mit dem Kohlenstoffatom von A verknüpft ist,
bilden kann,
B für N, C-H, C-F,
Y für Wasserstoff steht oder gemeinsam mit R² eine Brücke der Struktur -*S-NH- bilden kann, wobei das mit * markierte Atom für Y steht, und
T einen Rest der Formel 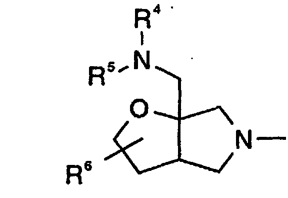 bedeutet, worin
R⁴ für H, CH₃, C₂H₅, gegebenenfalls durch Amino substituiertes Acyl mit 1 bis 4 Kohlenstoffatomen oder Alkoxycarbonyl mit 1 bis 4 Kohlenstoffatomen im Alkylteil,
R⁵ für H und
R⁶ für H stehen.

4. Verwendung gemäß Anspruch 1 von diastereomerenreinen und enantiomerenreinen Verbindungen nach den Ansprüchen 1 bis 3.

5. Verwendung gemäß Anspruch 1 von (+)-7-[(1S,5R)-1-Aminomethyl-2-oxa-7-aza-bicyclo[3.3.0]oct-7-yl]-8-cyano-1-cyclopropyl-6-fluor-1,4-dihydro-4-oxo-chinolincarbonsäure.

6. Diastereomerenreine und enantiomerenreine Verbindungen aus der Gruppe bestehend aus
7-(1-Aminomethyl-2-oxa-7-aza-bicyclo[3.3.0]oct-7-yl)-1-cyclopropyl-8-difluormethoxy-6-fluor-1,4-dihydro-4-oxo-3-chinolincarbonsäure und
7-(1-Aminomethyl-2-oxa-7-aza-bicyclo[3.3.0]oct-7-yl)-8-cyano-1-cyclopropyl-6-fluor-1,4-dihydro-4-oxo-3-chinolincarbonsäure.

7. (+)-7-[(1S,5R)-1-Aminomethyl-2-oxa-7-aza-bicyclo[3.3.0]oct-7-yl]-8-cyano-1-cyclopropyl-6-fluor-1,4-dihydro-4-oxo-chinolincarbonsäure.

8. (+)-7-[(1S,5R)-1-Aminomethyl-2-oxa-7-aza-bicyclo[3.3.0]oct-7-yl]-8-cyano-1-cyclopropyl-6-fluor-1,4-dihydro-4-oxo-chinolincarbonsäure zur Herstellung von Arzneimitteln.

9. Arzneimittel enthaltend (+)-7-[(1S,5R)-1-Aminomethyl-2-oxa-7-azabicyclo[3.3.0]oct-7-yl]-8-cyano-1-cyclopropyl-6-fluor-1,4-dihydro-4-oxochinolincarbonsäure.

## Claims

1. Use of compounds of the formula (I) 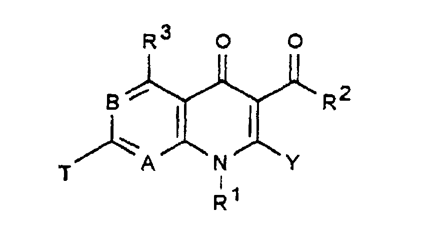 in which
R¹ represents alkyl having 1 to 4 carbon atoms which is optionally mono- to trisubstituted by halogen or hydroxyl, alkenyl having 2 to 4 carbon atoms, cycloalkyl having 3 to 6 carbon atoms which is optionally substituted by 1 or 2 fluorine atoms, bicyclo[1.1.1]pent-1-yl, 1,1-dimethylpropargyl, 3-oxetanyl, methoxy, amino, methylamino, dimethylamino, phenyl which is optionally mono-or disubstituted by halogen, amino or hydroxyl, isoxazolyl, thiadiazolyl,
R² represents hydroxyl, alkoxy having 1 to 4 carbon atoms which is optionally substituted by hydroxyl, methoxy, amino, dimethylamino or ethoxycarbonyl, benzyloxy, allyloxy, propargyloxy or (5-methyl-2-oxo-1,3-dioxol-4-yl)-methyloxy, acetoxymethyloxy, pivaloyloxymethyloxy, 5-indanyloxy, phthalidinyloxy, 3-acetoxy-2-oxo-butyloxy, nitromethyl or dialkoxycarbonylmethyl having 1 to 2 carbon atoms in each alkyl moiety,
R³ represents hydrogen, amino, hydroxyl, methyl or halogen,
A represents N or C-R⁷ in which
R⁷ represents hydrogen, halogen, CF₃, OCH₃, OCHF₂, CH₃, CN, CH=CH₂ or C≡CH or else together with R¹ may form a bridge of the structure -*O-CH₂-CH-CH₃, -*S-CH₂-CH₂-, -*S-CH₂-CH-CH₃, -*CH₂-CH₂-CH-CH₃ or -*O-CH₂-N-R⁸ where the atom marked with * is attached to the carbon atom of A and in which
R⁸ represents hydrogen, methyl or formyl,
B represents N, C-H, C-F, C-Cl, C-NO₂, C-NH₂,
Y represents hydrogen or together with R² may form a bridge of the structure -*S-NH- where the atom marked with * represents Y, and
T represents a radical of the formula 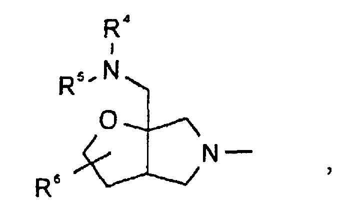 in which
R⁴ represents H, CH₃, C₂H₅, optionally amino-substituted acyl having 1 to 5 carbon atoms, alkoxycarbonyl, aminocarbonyl, alkylthio-thiocarbonyl and dialkoxyphosphoryl having 1 to 4 carbon atoms in the alkyl moiety,
R⁵ represents H, CH₃, C₂H₅ and
R⁶ represents H, CH₃,
and their pharmaceutically useful hydrates and acid addition salts and the alkali metal, alkaline earth metal, silver and guanidinium salts of the parent carboxylic acids for preparing medicaments for the therapy of Helicobacter pylori infections and associated gastroduodenal disorders.

2. Use according to Claim 1 in which in formula (I)
R¹ represents alkyl having 1 to 4 carbon atoms which is optionally mono- to trisubstituted by fluorine, represents vinyl, optionally fluorine-substituted cyclopropyl, bicyclo[1.1.1]pent-1-yl, 1,1-dimethylpropargyl, 3-oxetanyl, methylamino, phenyl which is optionally mono- or disubstituted by fluorine, amino or hydroxyl, thiadiazolyl,
R² represents hydroxyl, optionally ethoxycarbonyl-substituted alkoxy having 1 to 4 carbon atoms, benzyloxy, allyloxy, propargyloxy,
R³ represents hydrogen, amino, hydroxyl, methyl or fluorine,
A represents N or C-R⁷ in which
R⁷ represents hydrogen, halogen, CF₃, OCH₃, OCHF₂, CH₃, CN, CH=CH₂ or C≡CH or else together with R¹ may form a bridge of the structure -*O-CH₂-CH-CH₃ or -*O-CH₂-N-R⁸ where the atom marked with * is attached to the carbon atom of A and in which
R⁸ is hydrogen or methyl,
B represents N, C-H, C-F, C-Cl, C-NH₂,
Y represents hydrogen or together with R² may form a bridge of the structure -*S-NH- where the atom marked with * represents Y, and
T represents a radical of the formula 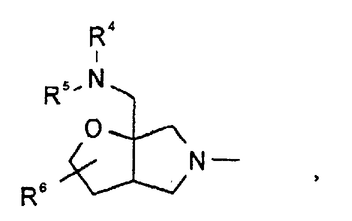 in which
R⁴ represents H, CH₃, C₂H₅, optionally amino-substituted acyl having 1 to 5 carbon atoms, alkoxycarbonyl, aminocarbonyl, alkylthio-thiocarbonyl and dialkoxyphosphoryl having 1 to 4 carbon atoms in the alkyl moiety,
R⁵ represents H, CH₃, C₂H₅ and
R⁶ represents H.

3. Use according to Claim 1 in which in formula (I)
R¹ represents alkyl having 1 to 4 carbon atoms which is optionally mono- or disubstituted by fluorine, optionally fluorine-substituted cyclopropyl, phenyl which is optionally mono-or disubstituted by fluorine,
R² represents hydroxyl, optionally ethoxycarbonyl-substituted alkoxy having 1 to 4 carbon atoms, benzyloxy, allyloxy, propargyloxy,
R³ represents hydrogen, amino, hydroxyl, methyl or fluorine,
A represents N or C-R in which
R⁷ represents hydrogen, chlorine, fluorine, OCH₃, OCHF₂, CH₃ or CN or else together with R¹ may form a bridge of the structure -*O-CH₂-CH-CH₃, or -*O-CH₂-N-CH₃ where the atom marked with * is attached to the carbon atom of A,
B represents N, C-H, C-F,
Y represents hydrogen or together with R² may form a bridge of the structure -*S-NH- where the atom marked with * represents Y, and
T represents a radical of the formula 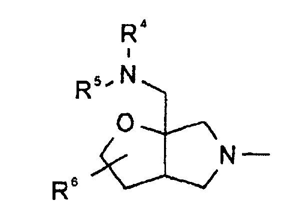 in which
R⁴ represents H, CH₃, C₂H₅, optionally amino-substituted acyl having 1 to 4 carbon atoms or alkoxycarbonyl having 1 to 4 carbon atoms in the alkyl moiety,
R⁵ represents H and
R⁶ represents H.

4. Use according to Claim 1 of diastereomerically pure and enantiomerically pure compounds according to Claims 1 to 3.

5. Use according to Claim 1 of (+)-7-[(1S,5R)-1-aminomethyl-2-oxa-7-aza-bicyclo[3.3.0]oct-7-yl]-8-cyano-1-cyclopropyl-6-fluoro-1,4-dihydro-4-oxo-quinoline-carboxylic acid.

6. Diastereomerically pure and enantiomerically pure compounds from the group consisting of
7-(1-aminomethyl-2-oxa-7-aza-bicyclo[3.3.0]oct-7-yl)-1-cyclopropyl-8-difluoromethoxy-6-fluoro-1,4-dihydro-4-oxo-3-quinolinecarboxylic acid, and
7-(1-aminomethyl-2-oxa-7-aza-bicyclo[3.3.0]oct-7-yl)-8-cyano-1-cyclopropyl-6-fluoro-1,4-dihydro-4-oxo-3-quinolinecarboxylic acid.

7. (+)-7-[(1S,5R)-1-Aminomethyl-2-oxa-7-aza-bicyclo[3.3.0]oct-7-yl]-8-cyano-1-cyclopropyl-6-fluoro-1,4-dihydro-4-oxo-quinolinecarboxylic acid.

8. (+)-7-[(1S,5R)-1-Aminomethyl-2-oxa-7-aza-bicyclo[3.3.0]oct-7-yl]-8-cyano-1-cyclopropyl-6-fluoro-1,4-dihydro-4-oxo-quinoline-carboxylic acid for preparing medicaments.

9. Medicaments comprising (+)-7-[(1S,5R)-1-aminomethyl-2-oxa-7-aza-bicyclo[3.3.0]oct-7-yl]-8-cyano-1-cyclopropyl-6-fluoro-1,4-dihydro-4-oxo-quinoline-carboxylic acid.

## Revendications

1. Utilisation de composés de formule (I) 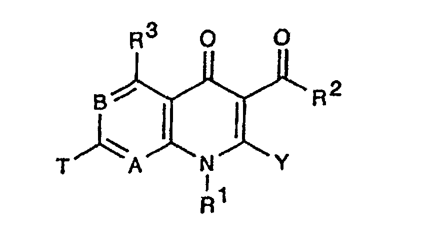 dans laquelle
R¹ représente un groupe alkyle ayant 1 à 4 atomes de carbone éventuellement substitué une à trois fois par un halogène ou un radical hydroxy, un groupe alcényle ayant 2 à 4 atomes de carbone, un groupe cycloalkyle ayant 3 à 6 atomes de carbone éventuellement substitués par 1 ou 2 atomes de fluor, un groupe bicyclo[1.1.1)pent-1-yle, 1,1-diméthylpropargyle, 3-oxétanyle, méthoxy, amino, méthylamino, diméthylamino, un groupe phényle éventuellement substitué par un halogène, un radical amino ou un radical hydroxy, un groupe isoxazolyle, thiadiazolyle,
R² représente un groupe hydroxy, un groupe alkoxy ayant 1 à 4 atomes de carbone éventuellement substitués par un radical hydroxy, méthoxy, amino, diméthylamino ou éthoxycarbonyle, un groupe benzyloxy, allyloxy, propargyloxy ou (5-méthyl-2-oxo-1,3-dioxole-4-yl)méthyloxy, acétoxyméthyloxy, pivaloyloxyméthyloxy, 5-indanyloxy, phtalidinyloxy, 3-acétoxy-2-oxobutyloxy, nitrométhyle ou dialkoxy-carbonylméthyle ayant 1 ou 2 atomes de carbone dans chaque partie alkyle,
R³ représente l'hydrogène, un groupe amino, hydroxy, méthyle ou un halogène,
A représente N ou un groupe C-R⁷, dans lequel
R⁷ représente l'hydrogène, un halogène, un radical CF₃, OCH₃, OCHF₂, CH₃, CN, CH=CH₂ ou C CH ou peut aussi former conjointement avec R¹ un pont de structure -*O-CH₂-CH-CH₃, -*S-CH₂-CH₂-, -*S-CH₂-CH-CH₃, -*CH₂-CH₂-CH-CH₃ ou -*O-CH₂-N-R⁸, dont l'atome marqué d'un * est lié à l'atome de carbone de A, et où
R⁸ représente l'hydrogène, un radical méthyle ou formyle,
ou
B représente N, C-H, C-F, C-Cl, C-NO₂, C-NH₂,
Y représente l'hydrogène ou forme conjointement avec R² un pont de structure -*S-NH-, où l'atome marqué d'un * représente Y, et
T est un reste de formule 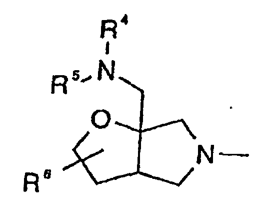
dans laquelle
R⁴ représente H, CH₃, C₂H₅, un groupe acyle ayant 1 à 5 atomes de carbone éventuellement substitués par un radical amino, un groupe alkoxycarbonyle, aminocarbonyle, alkylthiothiocarbonyle, et un groupe dialkoxyphosphoryle ayant 1 à 4 atomes de carbone dans la partie alkyle,
R⁵ représente H, CH₃, C₂H₅ et
R⁶ représente H, CH₃,
et leurs hydrates et leurs d'addition d'acides acceptables du point de vue pharmaceutique, de même que les sels de métaux alcalins, de métaux alcalinoterreux, d'argent et de guanidinium, des acides carboxyliques de base, pour la préparation de médicaments destinés au traitement d'infections dues à *Helicobacter pylori* et des affections gastroduodénales qui y sont associées.

2. Utilisation suivant la revendication 1, selon laquelle, dans la formule (I)
R¹ représente un groupe alkyle ayant 1 à 4 atomes de carbone éventuellement substitué une à trois fois par du fluor, un groupe vinyle, un groupe cyclopropyle éventuellement substitué par du fluor, un groupe bicyclo[1.1.1]pent-1-yle, 1,1-diméthylpropargyle, 3-oxétanyle, méthylamino, un groupe phényle éventuellement substitué une ou deux fois par du fluor, un radical amino ou hydroxy, un groupe thiadiazolyle,
R² représente un groupe hydroxy, un groupe alkoxy ayant 1 à 4 atomes de carbone éventuellement substitués par un radical éthoxycarbonyle, un groupe benzyloxy, allyloxy, propargyloxy,
R³ représente l'hydrogène, un groupe amino, hydroxy, méthyle ou le fluor,
A représente N ou un groupe C-R⁷, dans lequel
R⁷ représente l'hydrogène, un halogène, un groupe CF₃, OCH₃, OCHF₂, CH₃, CN, CH=CH₂ ou C CH ou peut aussi former conjointement avec R¹ un pont de structure -*O-CH₂-CH-CH₃ ou -*O-CH₂-N-R⁸, dont l'atome marqué d'un * est lié à l'atome de carbone de A, et où
R⁸ représente l'hydrogène ou un groupe méthyle,
B représente N, C-H, C-F, C-Cl, C-NH₂,
Y représente l'hydrogène ou peut former conjointement avec R² un pont de structure -*S-NH-, où l'atome marqué d'un * représente Y, et
T est un reste de formule 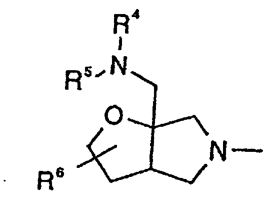
dans laquelle
R⁴ représente H, CH₃, C₂H₅, un groupe acyle ayant 1 à 5 atomes de carbone éventuellement substitués par un radical amino, des groupes alkoxycarbonyle, aminocarbonyle, alkylthiothiocarbonyle, et dialkoxyphosphoryle ayant 1 à 4 atomes de carbone dans la partie alkyle,
R⁵ représente H, CH₃, C₂H₅ et
R⁶ représente H.

3. Utilisation suivant la revendication 1, selon laquelle, dans la formule (I)
R¹ représente un groupe alkyle ayant 1 à 4 atomes de carbone éventuellement substitué une ou deux fois par du fluor, un groupe cyclopropyle éventuellement substitué par du fluor, un groupe phényle éventuellement substitué une ou deux fois par du fluor,
R² représente un groupe hydroxy, un groupe alkoxy ayant 1 à 4 atomes de carbone éventuellement substitués par un radical éthoxycarbonyle, un groupe benzyloxy, allyloxy, propargyloxy,
R³ représente l'hydrogène, un groupe amino, hydroxy, méthyle ou le fluor,
A représente N ou un groupe C-R⁷, dans lequel
R⁷ représente l'hydrogène, le chlore, le fluor, un groupe OCH₃, OCHF₂, CH₃ ou CN ou peut aussi former conjointement avec R¹ un pont de structure -*O-CH₂-CH-CH₃, -*O-CH₂-N-CH₃, dont l'atome marqué d'un * est lié à l'atome de carbone de A,
B représente N, C-H, C-F,
Y représente l'hydrogène ou peut former conjointement avec R² un pont de structure -*S-NH-, dont l'atome marqué d'un * représente Y, et
T est un reste de formule 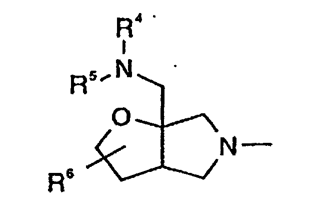 dans laquelle
R⁴ représente H, CH₃, C₂H₅, un groupe acyle ayant 1 à 4 atomes de carbone éventuellement substitués par un radical amino ou un groupe alkoxycarbonyle ayant 1 à 4 atomes de carbone dans la partie alkyle,
R⁵ représente H, et
R⁶ représente H.

4. Utilisation suivant la revendication 1 de composés diastéréo-isomères purs et énantiomères purs selon les revendications 1 à 3.

5. Utilisation selon la revendication 1 de l'acide (+)-7-[(1S,5R)-1-aminométhyl-2-oxa-7-azabicyclo[3.3.0]oct-7-yl]-8-cyano-1-cyclopropyl-6-fluoro-1,4-dihydro-4-oxoquinoléinecarboxylique.

6. Composés diastéréo-isomères purs et énantiomères purs du groupe comprenant :
l'acide 7-(1-aminométhyl-2-oxa-7-azabicyclo[3.3.0]oct-7-yl)-1-cyclopropyl-8-difluorométhoxy-6-fluoro-1,4-dihydro-4-oxo-3-quinoléinecarboxylique et
l'acide 7-(1-aminométhyl-2-oxa-7-azabicyclo[3.3.0]oct-7-yl)-8-cyano-1-cyclopropyl-6-fluoro-1,4-dihydro-4-oxo-3-quinoléinecarboxylique.

7. L'acide (+)-7-[(1S,5R)-1-aminométhyl-2-oxa-7-azabicyclo[3.3.0]oct-7-yl]-8-cyano-1-cyclopropyl-6-fluoro-1,4-dihydro-4-oxoquinoléinecarboxylique.

8. L'acide (+)-7-[(1S,5R)-1-aminométhyl-2-oxa-7-azabicyclo[3.3.0]oct-7-yl]-8-cyano-1-cyclopropyl-6-fluoro-1,4-dihydro-4-oxoquinoléinecarboxylique destiné à la préparation de médicaments.

9. Médicaments contenant de l'acide (+)-7-[(1S,5R)-1-aminométhyl-2-oxa-7-azabicyclo[3.3.0]oct-7-yl]-8-cyano-1-cyclopropyl-6-fluoro-1,4-dihydro-4-oxoquinoléinecarboxylique.
